# EUROPEAN PATENT APPLICATION

(11) **EP 4 506 468 A1**
(43) Date of publication of application: **12.02.2025**
(21) Application number: 23382839.1
(22) Date of filing: 11.08.2023
(51) Int. Cl.: C12Q 1/6883, G01N 33/53

(54) **METHOD FOR DIAGNOSING COGNITIVE IMPAIRMENT THROUGH ADIPOSE TISSUE / BLOOD AND COMPOUNDS USEFUL FOR ITS TREATMENT**

(71) Applicant: Fundació Institut d'Investigació Biomèdica de Girona Dr. Josep Trueta (IDIBGI), 17190 Salt (Girona) (ES); Universitat Pompeu Fabra, 08002 Barcelona (ES)
(72) Inventor: Mayneris Perxachs, Jordi, Salt (GIRONA) (ES); Fernández-Real Lemos, José Manuel, Salt (GIRONA) (ES); Castells Nobau, Anna, Salt (GIRONA) (ES); Martin-Garcia, Elena, Barcelona (ES); Maldonado, Rafael, Barcelona (ES)
(74) Representative: Pons

(57) **Abstract**

The present invention relates to a method for *in vitro* method for diagnosing a cognitive impairment in a subject, or to *in vitro* method for monitoring the response to a therapy directed to improve the cognitive function in a subject suffering from cognitive impairment, comprising determining the expression levels of a gene specifically expressed in adipose tissue. The invention also discloses a kit to into practice the methods of the invention as well as a composition for use in the treatment of cognitive impairment by targeting the altered expression of this gene in adipose tissue.

## Description

The present invention relates to *in vitro* method for diagnosing a cognitive impairment by determining the expression levels of a gene specifically expressed in adipose tissue and/or blood, as well as composition for the treatment of cognitive impairment by targeting the altered expression of this gene in adipose tissue.

### BACKGROUND ART

There is an increased awareness that peripheral tissues modulate brain function shaping different cognitive domains. Recent studies in rodents revealed that restoring glucose metabolism of myeloid cells reversed cognitive decline in ageing, whereas selective gastrointestinal vagal afferent ablation impaired hippocampus-dependent episodic and spatial memory in rats. The liver has also been proposed to play a major role in regulating feeding behaviour in mice.

Obesity is associated with increased risk of cognitive impairment. An increased fatness in middle-aged adults (<65y) predict decreased cognitive abilities in late life, mainly memory, executive functioning and learning (6-8). However, the precise mechanisms involved remain largely unknown.

The brain regulates adipose tissue performance. For instance, sympathetic neuro-adipose connections mediate lipolysis and cytokine production while neuroimmune interactions are involved in thermoregulation. Recent observations in rodents show that adipose tissue also regulates brain function. Genetically-modified mice engineered to release the peptide NaKtide in adipocytes led to improved hippocampal memory through the inhibition of Na,K-ATPase signalling in adipocytes. Visceral adipose NLRP3 was linked to impaired memory in obese mice via IL-1R1 on CX3CR1+ cells, leading to microglial activation. An increased abundance of beige adipocytes in subcutaneous fat restored hippocampal synaptic plasticity in mice through induction of anti-inflammatory cytokine IL-4, protecting from obesity-induced cognitive impairment.

There is a necessity in the state to the art to provide methods for diagnosing cognitive impairment.

### DESCRIPTION OF THE INVENTION

The inventors have identified 188 genes from RNA sequencing of adipose tissue (AT) in three cohorts that were associated with performance in different cognitive domains. These genes were mostly involved in synaptic function, phosphatidylinositol metabolism, the complement cascade, anti-inflammatory signaling, and vitamin metabolism. These findings were translated into the plasma metabolome. The circulating blood expression levels of most of these genes were also associated with several cognitive domains in a cohort of 816 subjects. Among them, downregulation of the neurotransmitter release cycle-associated gene *SLC18A2* improved cognitive abilities in *Drosophila* and in mice. Upregulation of *RIMS1* in *Drosophila* fat body enhanced cognitive abilities. The present invention shows novel connections between AT transcriptome and brain function in humans, providing unprecedented diagnostic/therapeutic targets in AT.

Thus, in one aspect, the present invention relates to an *in vitro* method for diagnosing a cognitive impairment in a subject, hereinafter "diagnosis method of the invention", comprising
(i) determining the expression levels of a gene specifically expressed in adipose tissue, or of a fragment thereof, in a biological sample isolated from the subject, wherein
   ▪ said gene is:
      - SLC18A2 (Solute Carrier Family 18 Member A2) gene, or
      - RIMS1 (Regulating Synaptic Exocytosis 1) gene, or
      - NUDT2 (Nudix hydrolase 2) gene, or
      - AMPH (Amphiphysin) gene, or
      - UNC5B (Unc-5 netrin receptor B) gene, or
      - OAT (Ornithine Aminotransferase) gene, or
      - EZR (Ezrin) gene, or
      - NR4A2 (Nuclear Receptor subfamily 4, group A, member 2) gene, and
   ▪ the biological sample isolated from the subject is blood or adipose tissue biopsy; and
(ii) comparing the expression levels obtained in (i) with the expression levels of said gene in a control sample, wherein
   - If the expression levels of SLC18A2 gene are increase with respect to the ones in the control sample, then the subject suffers from a cognitive impairment; or
   - If the expression levels of RIMS1 gene are decrease with respect to the ones in the control sample, then the subject suffers from a cognitive impairment; or
   - If the expression levels of NUDT2 gene are decrease with respect to the ones in the control sample, then the subject suffers from a cognitive impairment with respect to the ones in the control sample, then the subject suffers from a cognitive impairment; or
   - If the expression levels of AMPH gene are decrease with respect to the ones in the control sample with respect to the ones in the control sample, then the subject suffers from a cognitive impairment; or
   - If the expression levels of UNC5B gene are decrease with respect to the ones in the control sample with respect to the ones in the control sample, then the subject suffers from a cognitive impairment; or
   - If the expression levels of OAT gene are decrease with respect to the ones in the control sample with respect to the ones in the control sample, then the subject suffers from a cognitive impairment; or
   - If the expression levels of EZR gene are decrease with respect to the ones in the control sample with respect to the ones in the control sample, then the subject suffers from a cognitive impairment; or
   - If the expression levels of NR4A2 gene are decrease with respect to the ones in the control sample with respect to the ones in the control sample, then the subject suffers from a cognitive impairment.

As use herein, the term "diagnosis" refers to the procedure by which a certain disease, nosological entity, syndrome, or any health-disease condition is identified by analyzing a series of clinical parameters or symptoms characteristic of said disease, and which distinguish it from other diseases with similar clinical pictures. By "diagnosis" is also intended to refer to the providing of information useful for diagnosis

As use herein, the term "cognitive impairment" refers to a person has trouble remembering, learning new things, concentrating, or making decisions that affect their everyday life. Cognitive impairment ranges from mild to severe. With mild impairment, people may begin to notice changes in cognitive functions, but still be able to do their everyday activities. Severe levels of impairment can lead to losing the ability to understand the meaning or importance of something and the ability to talk or write, resulting in the inability to live independently. A few commons signs of cognitive impairment include, without limiting to, bemory loss, frequently asking the same question or repeating the same story over and over, not recognizing familiar people and places, having trouble exercising judgment, such as knowing what to do in an emergency, changes in mood or behavior, vision problems, difficulty planning and carrying out tasks. In a particular embodiment of the diagnosis method of the invention, the cognitive impairment is Mild Cognitive Impairment (MCI), Age-related Cognitive Decline (ARCD) or Age-Associated Memory Impairment (AAMI).

The present invention can be applied to any subject. As used herein, the term "subject" is equivalent to the term "individual", whereby both terms can be used interchangeably throughout the present description. "Subject" means any animal belonging to any species. However, in a particular embodiment of the diagnosis method of the invention, alone or in combination with the previous particular embodiments, the subject is a mammal, preferably a primate, more preferably a human being of any sex or age.

In another particular embodiment, the subject is subject having obesity or overweight. As used herein, the term "obesity" or "overweight" in general refers to an abnormal or excessive fat accumulation that presents a risk to health, being "overweight" a level of obesity. A crude population measure of obesity in adults is the body mass index (BMI), a person's weight (in kilograms) divided by the square of his or her height (in meters). The term "obesity" is herein adopted to describe a condition characterized by, preferably, a BMI ≥ 30 kg/m² while the term "overweight" is herein adopted to describe a condition characterized by, preferably, a BMI ≥ 25 kg/m² but < 30 kg/m². For adolescents, "obesity" refers to a condition characterized by two standard deviations body mass index for age and sex from the World Health Organization (WHO) growth reference for school-aged children and adolescents. The terms "obesity" and "overweight" thus imply a medical indication for treatment. In a particular embodiment of the diagnosis method of the invention, alone or in combination with all or each one of the previous particular embodiments, the subject is a human subject having a body Mass Index (BMI) equal or higher than 25 kg/m², preferably, equal or higher than 30 Kg/m², more preferably, equal or higher than 35 Kg/m², still more preferably, equal or higher than 40 Kg/m².

The diagnosis method of the invention comprises a first step of determining the expression levels of a gene specifically expressed in adipose tissue, or of a fragment thereof, in a biological sample isolated from the subject,
wherein
▪ said gene is:
   - SLC18A2 gene, or
   - RIMS1 gene, or
   - NUDT2 gene, or
   - AMPH gene, or
   - UNC5B gene, or
   - OAT gene, or
   - EZR gene, or
   - NR4A2 gene, and
▪ the biological sample isolated from the subject is plasma, blood or adipose tissue.

As use herein, the expression "a gene specifically expressed in adipose tissue" refers to gene which is specifically expressed by the adipocytes.

As use herein, the term "expression levels of a gene" refers to the amount of the molecules resulting from the transcription and/or translation of said gene, i.e the cDNA, mRNA or protein. Any of these molecules can be used for determining the expression levels of a gene. The diagnosis method of the invention also encompasses measuring the expression levels of a fragment of the gen, i.e. a cDNA fragment, mRNA fragment or a protein fragment coming from the transcription and/or translation of the gene.

Thus, in a particular embodiment of the diagnosis method of the invention, the determination of the expression levels of the gene comprises measuring or qualifying the level of cDNA, or a fragment thereof, the level of mRNA, or a fragment thereof, and/or the level of the protein encoded by said gene, or a fragment thereof.

As used herein, "mRNA fragment" or "cDNA fragment" is understood as the nucleotide sequence of a gene comprising one or more nucleotides absent from the 3' and/or 5' ends with respect to the complete nucleotide sequence of the gene.

Similarly, in the present invention, "fragment of a protein" or "protein fragment" is understood as the amino acid sequence of the protein comprising one or more amino acids absent from the terminal amino end or terminal carboxyl end thereof with respect to the complete amino acid sequence of the protein.

The diagnosis method of the invention comprises determining the expression levels of a gene specifically expressed in adipose tissue, wherein said gene include, without limiting to, the SLC18A2 gene, the RIMS1 gene, the NUDT2 gene, the AMPH gene, the UNC5B gene, the OAT gen, the EZR gene, and the NR4A2 gene.

The SLC18A2 (Solute Carrier Family 18 Member A2) gene, is a gene that, in humans, is located on the chromosome 10q25.3. Alternative names that can be used in the scientific literature to refer to the SLC18A2 gene include, without limiting to, PKDYS2, SVAT, SVMT, VAT2, VMAT2. In a particular embodiment of the diagnosis method of the invention, alone or in combination with all or each one of the previous particular embodiments, the SLC18A2 gene comprises, or consists of, a nucleotide sequence with a sequence identity of, at least, 80, 85, 90, 95, 96, 97, 98, 99% with the nucleotide sequence SEQ ID NO: 1 (NCBI Reference Sequence: NM_003054.6). In another particular embodiment, the SLC18A2 gene comprises, or consists of, a nucleotide sequence with a sequence identity of 100% with the nucleotide sequence SEQ ID NO: 1.

The SLC18A2 gene encodes the synaptic vesicular amine transporter protein. In a particular embodiment of the diagnosis method of the invention, alone or in combination with all or each one of the previous particular embodiments, the protein encoded by the SLC18A2 gene comprises, or consists of, an amino acid sequence with a sequence identity of, at least, 80, 85, 90, 95, 96, 97, 98, 99% with the sequence SEQ ID NO: 2 (NCBI Reference Sequence: NP_003045.2). In another particular embodiment of the diagnosis method of the invention, alone or in combination with all or each one of the previous particular embodiments, the protein encoded by the SLC18A2 gene comprises, or consists of, an amino acid sequence with a sequence identity of 100% with the sequence SEQ ID NO: 2.

The RIMS1 (Regulating Synaptic Exocytosis 1) gene, is a gene that, in humans, is located on the 6q13 chromosome. Alternative names that can be used in the scientific literature to refer to the RIMS1 gene include, without limiting to, CORD7, RAP3IP2, RIM, RIM1. In a particular embodiment of the diagnosis method of the invention, alone or in combination with all or each one of the previous particular embodiments, the RIMS1 gene comprises, or consists of, a nucleotide sequence with a sequence identity of, at least, 80, 85, 90, 95, 96, 97, 98, 99% with the nucleotide sequence SEQ ID NO: 3 (NCBI reference number NM_001168407.2). In another particular embodiment, the RIMS1 gene comprises, or consists of, a nucleotide sequence with a sequence identity of 100% with the nucleotide sequence SEQ ID NO: 3.

The RIMS1 gene encodes the synaptic vesicle exocytosis protein. In a particular embodiment of the diagnosis method of the invention, alone or in combination with all or each one of the previous particular embodiments, the protein encoded by the RIMS1 gene comprises, or consists of, an amino acid sequence with a sequence identity of, at least, 80, 85, 90, 95, 96, 97, 98, 99% with the sequence SEQ ID NO: 4 (NCBI reference number NP_001161879.1). In another particular embodiment of the diagnosis method of the invention, alone or in combination with all or each one of the previous particular embodiments, the protein encoded by the RIMS1 gene comprises, or consists of, an amino acid sequence with a sequence identity of 100% with the sequence SEQ ID NO: 4.

The NUDT2 (Nudix hydrolase 2) gene, is a gene that, in humans, is located on the chromosome 9p13.3. Alternative names that can be used in the scientific literature to refer to the NUDT2 gene include, without limiting to, APAH1 and IDDPN. In a particular embodiment of the diagnosis method of the invention, alone or in combination with all or each one of the previous particular embodiments, the NUDT2 gene comprises, or consists of, a nucleotide sequence with a sequence identity of, at least, 80, 85, 90, 95, 96, 97, 98, 99% with the nucleotide sequence SEQ ID NO: 5 (NCBI reference number NM_001161.5). In another particular embodiment, the NUDT2 gene comprises, or consists of, a nucleotide sequence with a sequence identity of 100% with the nucleotide sequence SEQ ID NO: 5.

The NUDT2 gene encodes the bis(5'-nucleosyl)-tetraphosphatase [asymmetrical] protein. In a particular embodiment of the diagnosis method of the invention, alone or in combination with all or each one of the previous particular embodiments, the protein encoded by the NUDT2 gene comprises, or consists of, an amino acid sequence with a sequence identity of, at least, 80, 85, 90, 95, 96, 97, 98, 99% with the sequence SEQ ID NO: 6 (NCBI reference number NP_001152.1). In another particular embodiment of the diagnosis method of the invention, alone or in combination with all or each one of the previous particular embodiments, the protein encoded by the NUDT2 gene comprises, or consists of, an amino acid sequence with a sequence identity of 100% with the sequence SEQ ID NO: 6.

The AMPH (Amphiphysin) gene, is a gene that, in humans, is located on the 7p14.1 chromosome. In a particular embodiment of the diagnosis method of the invention, alone or in combination with all or each one of the previous particular embodiments, the AMPH gene comprises, or consists of, a nucleotide sequence with a sequence identity of, at least, 80, 85, 90, 95, 96, 97, 98, 99% with the nucleotide sequence SEQ ID NO: 7 (NCBI reference number NM_001635.4). In another particular embodiment, the AMPH gene comprises, or consists of, a nucleotide sequence with a sequence identity of 100% with the nucleotide sequence SEQ ID NO: 7.

The AMPH gene encodes by a protein associated with the synaptic vesicles present in the cytoplasmic surface. In a particular embodiment of the diagnosis method of the invention, alone or in combination with all or each one of the previous particular embodiments, the protein encoded by the AMPH gene comprises, or consists of, an amino acid sequence with a sequence identity of, at least, 80, 85, 90, 95, 96, 97, 98, 99% with the sequence SEQ ID NO: 8 (NCBI reference number NP_001626.1). In another particular embodiment of the diagnosis method of the invention, alone or in combination with all or each one of the previous particular embodiments, the protein encoded by the AMPH gene comprises, or consists of, an amino acid sequence with a sequence identity of 100% with the sequence SEQ ID NO: 8.

The UNC5B (Unc-5 netrin receptor B) gene, is a gene that, in humans, is located on the 10q22.1 chromosome. Alternative names that can be used in the scientific literature to refer to the UNC5B gene include, without limiting to, UNC5H2 and p53RDL1. In a particular embodiment of the diagnosis method of the invention, alone or in combination with all or each one of the previous particular embodiments, the UNC5B gene comprises, or consists of, a nucleotide sequence with a sequence identity of, at least, 80, 85, 90, 95, 96, 97, 98, 99% with the nucleotide sequence SEQ ID NO: 9 (NCBI reference number NM_001244889.2). In another particular embodiment, the A UNC5B gene comprises, or consists of, a nucleotide sequence with a sequence identity of 100% with the nucleotide sequence SEQ ID NO: 9.

The UNC5B gene encodes the netrin receptor UNC5B precursor protein. In a particular embodiment of the diagnosis method of the invention, alone or in combination with all or each one of the previous particular embodiments, the protein encoded by the UNC5B gene comprises, or consists of, an amino acid sequence with a sequence identity of, at least, 80, 85, 90, 95, 96, 97, 98, 99% with the sequence SEQ ID NO: 10 (NCBI reference number NP_001231818.1). In another particular embodiment of the diagnosis method of the invention, alone or in combination with all or each one of the previous particular embodiments, the protein encoded by the UNC5B gene comprises, or consists of, an amino acid sequence with a sequence identity of 100% with the sequence SEQ ID NO: 10.

The OAT (Ornithine Aminotransferase) gene, is a gene that, in humans, is located on the 10q26.13 chromosome. Alternative names that can be used in the scientific literature to refer to the OAT gene include, without limiting to, OKT, GACR, HOGA and OATASE. In a particular embodiment of the diagnosis method of the invention, alone or in combination with all or each one of the previous particular embodiments, the OAT gene comprises, or consists of, a nucleotide sequence with a sequence identity of, at least, 80, 85, 90, 95, 96, 97, 98, 99% with the nucleotide sequence SEQ ID NO: 11 (NCBI reference number NM_000274.4). In another particular embodiment, the OAT gene comprises, or consists of, a nucleotide sequence with a sequence identity of 100% with the nucleotide sequence SEQ ID NO: 11.

The OAT gene encodes the mitochondrial enzyme ornithine aminotransferase that converts arginine and ornithine into the major excitatory and inhibitory neurotransmitters glutamate and GABA. In a particular embodiment of the diagnosis method of the invention, alone or in combination with all or each one of the previous particular embodiments, the protein encoded by the OAT gene comprises, or consists of, an amino acid sequence with a sequence identity of, at least, 80, 85, 90, 95, 96, 97, 98, 99% with the sequence SEQ ID NO: 12 (NCBI reference number NP_000265.1). In another particular embodiment of the diagnosis method of the invention, alone or in combination with all or each one of the previous particular embodiments, the protein encoded by the OAT gene comprises, or consists of, an amino acid sequence with a sequence identity of 100% with the sequence SEQ ID NO: 12.

The EZR (Ezrin) gene, is a gene that, in humans, is located on the 6p25.3 chromosome. Alternative names that can be used in the scientific literature to refer to the EZR gene include, without limiting to, CVL, CVIL, VIL2 and HEL-S-105. In a particular embodiment of the diagnosis method of the invention, alone or in combination with all or each one of the previous particular embodiments, the EZR gene comprises, or consists of, a nucleotide sequence with a sequence identity of, at least, 80, 85, 90, 95, 96, 97, 98, 99% with the nucleotide sequence SEQ ID NO: 13 (NCBI reference number NM_001111077.2). In another particular embodiment, the EZR gene comprises, or consists of, a nucleotide sequence with a sequence identity of 100% with the nucleotide sequence SEQ ID NO: 13.

The EZR gene encodes the ezrin protein. In a particular embodiment of the diagnosis method of the invention, alone or in combination with all or each one of the previous particular embodiments, the protein encoded by the EZR gene comprises, or consists of, an amino acid sequence with a sequence identity of, at least, 80, 85, 90, 95, 96, 97, 98, 99% with the sequence SEQ ID NO: 14 (NCBI reference number NP_001104547.1) In another particular embodiment of the diagnosis method of the invention, alone or in combination with all or each one of the previous particular embodiments, the protein encoded by the EZR gene comprises, or consists of, an amino acid sequence with a sequence identity of 100% with the sequence SEQ ID NO: 14.

The NR4A2 (Nuclear Receptor subfamily 4, group A, member 2) gene, is a gene that, in humans, is located on the 2q24.1 chromosome. Alternative names that can be used in the scientific literature to refer to the NR4A2 gene include, without limiting to, NOT, RNR1, HZF-3, IDLDP, NURR1 and TINUR. In a particular embodiment of the diagnosis method of the invention, alone or in combination with all or each one of the previous particular embodiments, the NR4A2 gene comprises, or consists of, a nucleotide sequence with a sequence identity of, at least, 80, 85, 90, 95, 96, 97, 98, 99% with the nucleotide sequence SEQ ID NO: 15 (NCBI reference number NM_006186.4). In another particular embodiment, the NR4A2 gene comprises, or consists of, a nucleotide sequence with a sequence identity of 100% with the nucleotide sequence SEQ ID NO: 15.

The NR4A2 gene encodes a member of the steroid-thyroid hormone-retinoid receptor superfamily protein. In a particular embodiment of the diagnosis method of the invention, alone or in combination with all or each one of the previous particular embodiments, the protein encoded by the NR4A2 gene comprises, or consists of, an amino acid sequence with a sequence identity of, at least, 80, 85, 90, 95, 96, 97, 98, 99% with the sequence SEQ ID NO: 16 (NCBI reference number NP_006177.1). In another particular embodiment of the diagnosis method of the invention, alone or in combination with all or each one of the previous particular embodiments, the protein encoded by the NR4A2 gene comprises, or consists of, an amino acid sequence with a sequence identity of 100% with the sequence SEQ ID NO: 16.

In the present invention, "sequence identity" is understood as the degree of similarity between two nucleotide (or amino acid) sequences obtained by aligning the two sequences. A degree of identity expressed as a percentage will be obtained based on the number of common residues among the aligned sequences. The degree of identity between two nucleotide (or amino acid) sequences can be determined by conventional methods with standard sequence alignment algorithms known in the state of the art, such as BLAST. The BLAST programs, for example, BLASTN, BLASTX and TBLASTX, BLASTP and TBLASTN are a part of the public domain on The National Center for Biotechnology Information (NCBI) website.

If quantification of the expression of the gene specifically expressed in adipose tissue is going to be carried out from the cDNA or the mRNA, first it is necessary to extract the nucleic acid from the isolated biological sample of the subject. For this purpose, the biological sample can be treated to physically or mechanically separate the structure of the tissue or the cell, freeing the intracellular components in an aqueous or organic solution in order to isolate and prepare the nucleic acids. The nucleic acids are extracted by means of methods known by the person skilled in the art and commercially available.

Once the nucleic acid is removed, the quantification of the expression of the gene is then carried out. Practically any conventional method can be used within the framework of the present invention to quantify the mRNA levels of the gene specifically expressed in adipose tissue or of the corresponding cDNA thereof. By way of illustration and not limitation, the levels of mRNA of said gene can be quantified by means of using conventional methods, for example, methods comprising the amplification of mRNA and the quantification of the product of the amplification of said mRNA, such as electrophoresis and staining, or alternatively, by means of Southern blot and the use of suitable probes, Northern blot and the use of specific probes of mRNA of the gene of interest (PTGDR gene) or of the corresponding cDNA thereof, S1 nuclease mapping, RT-PCR, hybridisation, microassays, etc.

Similarly, the levels of the cDNA corresponding to said mRNA of the gene specifically expressed in adipose tissue can also be quantified by means of the use of conventional techniques: in this case, the diagnosis method of the invention includes a synthesis phase of the corresponding cDNA by means of reverse transcription (RT) of the corresponding mRNA followed by amplification and quantification of the product of the amplification of said cDNA. Conventional methods for quantifying the expression levels are widely known in the state of the art. In a particular embodiment of the diagnosis method of the invention, alone or in combination with all or each one of the previous particular embodiments, the quantification of the gene expression levels is carried out by means of a chain reaction of the quantitative polymerase (PCR), a DNA or RNA array, or RNA-Seq or Massive Sequencing applied to the study of RNA.

If the quantification of the expression levels of the gene specifically expressed in adipose tissue is going to be carried out from the protein encoded by the gen, then the isolated biological sample of the subject must be treated to extract the proteins. Methods for extracting or isolating proteins are known by the person skilled in the art and they are commercially available.

The levels of protein can be quantified by means of any conventional method that enables said protein to be detected and quantified in a sample of a subject. By way of illustration and not limitation, the levels of said protein can be quantified, for example, by means of the use of antibodies with the capacity to specifically recognize and bind to protein and the subsequent quantification of the complexes formed.

As used herein, the term "Antibody" is understood as a glycoprotein of the gamma globulin type that forms part of the humoral immune system that specifically binds to an antigen. The term antibody, as herein used, includes monoclonal antibodies, polyclonal antibodies, recombinant antibody fragments, combibodies, Fab and scFv antibody fragments, as well as the ligand binding domains. Specific antibodies of the protein encoded by the PGC-1alpha gene are commercially available. Likewise, the methods for producing antibodies are widely known in the state of the art.

The antibodies used in these assays can be labelled or non-labelled. The terms "label" or "labelled" refer to a composition capable of producing an indicative and detectable signal of the presence of the labelled molecule. Illustrative examples of suitable labels include, but are not limited to, radioisotopes, nucleotide chromophores, enzymes, substrates, fluorescent molecules, chemiluminescent remains, magnetic particles, bioluminescent remains, and similar. As such, a label is any composition that can be detected by spectroscopic, photochemical, biochemical, immunochemical, electrical, optical or chemical means. There is a wide range of known assays that can be used in the present invention, which use non-labeled antibodies (primary antibody) and labeled antibodies (secondary antibody); these techniques include Western-blot or Western transfer, ELISA (enzyme-linked immunosorbent assay), RIA (radioimmuneassay), DAS-ELISA (double antibody sandwich ELISA), immunocytochemical and immunohistochemical techniques, techniques based on the use of protein biochips or microarrays that include specific antibodies or assays based on colloidal precipitation in formats such as dipsticks. Other ways of detecting and quantifying the PPAR gamma coactivator-1 protein include affinity chromatography techniques, ligand binding assays, mass spectrometry, etc. However, in a particular embodiment, the quantification of the protein levels is carried out by means of Western blot, ELISA, a protein array or a binding study. When an immunological method is used, any antibody or reagent that is known to bind to the PPAR gamma coactivator-1 protein with high affinity can be used to detect the quantity thereof. Examples of antibodies or reagents with the capacity to bind to said proteins encoded by genes specifically expressed in adipose tissue include, without being limited to, polyclonal sera, hybridoma supernatants or monoclonal antibodies, antibody fragments, Fv, Fab, Fab' and F(ab')2, scFv, diabodies, triabodies, tetrabodies and humanised antibodies. On the market there are commercial antibodies against said proteins that can be used in the context of the present invention.

As explained in the preceding paragraphs, to carry out the first step of the diagnosis method of the invention, it is necessary to have an isolated biological sample of the subject. In the context of the present invention, the term "biological sample" relates to any biological that can be obtained from the individual, such as a biopsy, a tissue, a cell or a fluid (serum, saliva, semen, sputum, tears, mucus, sweat, milk, brain extracts, and similar), and that can store information about the characteristic genetic makeup of an individual. In a particular embodiment, the biological sample is blood, serum, plasma o tissue from a biopsy. The term "isolated" implies that the biological sample has been separated or extracted from the rest of the components that naturally accompany it. Techniques for obtaining biological samples of an individual are widely known in the state of the art, and any of them can be used in implementing the present invention.

Once first step of the diagnosis method of the invention has been carried out, i.e. the expression levels of the gene specifically expressed in adipose tissue have been quantified, the method comprises comparing the obtained expression levels to the corresponding expression levels of the gen in a control sample. As use herein, the term "control sample" refers to sample obtained from a subject of similar in age, sex, BMI and years of education-adjusted normal cognition that the subject to be diagnosed. The term "similar" means that both subjects are equivalent or comparable in the parameters of age, sex, BMI and years of education-adjusted normal cognition. It is routine practice for the skilled person in the technical field of the present invention, to evaluate the cognitive function in a subject by means of tests widely known in the state of the art, being common general knowledge the election of the subject which are going to be used to obtained the control sample.

Therefore, after comparing the expression levels of the gene can be concluded that
- If the expression levels of SLC18A2 gene are increase with respect to the ones in the control sample, then the subject suffers from a cognitive impairment; or
- If the expression levels of RIMS1 gene are decrease with respect to the ones in the control sample, then the subject suffers from a cognitive impairment; or
- If the expression levels of NUDT2 gene are decrease with respect to the ones in the control sample, then the subject suffers from a cognitive impairment; or
- If the expression levels of AMPH gene are decrease with respect to the ones in the control sample, then the subject suffers from a cognitive impairment; or
- If the expression levels of UNC5B gene are decrease with respect to the ones in the control sample, then the subject suffers from a cognitive impairment; or
- If the expression levels of OAT gene are decrease with respect to the ones in the control sample, then the subject suffers from a cognitive impairment; or
- If the expression levels of EZR gene are decrease with respect to the ones in the control sample, then the subject suffers from a cognitive impairment; or
- If the expression levels of NR4A2 gene are decrease with respect to the ones in the control sample, then the subject suffers from a cognitive impairment.

In the present invention, it is understood that an expression level are "increased" with respect to another when the expression levels of the gene are, at least, 1.5 times, than the expression levels of the gene in the control sample. Likewise, it is understood that an expression level is "decreased" with respect to another one when the expression levels of the gene are, at least, 1.5 times, or even more, than the expression levels of the gene in the control sample.

In another aspect, the present invention relates to an *in vitro* method for monitoring the response to a therapy directed to improve the cognitive function in a subject suffering from cognitive impairment, hereinafter "monitoring method of the invention", comprising
(i) determining the expression levels of a gene specifically expressed in adipose tissue, or of a fragment thereof, in a biological sample isolated from the subject after the administration of the therapy to the subject, wherein
   ▪ said gene is SLC18A2 gene, RIMS1 gene, NUDT2 gene, AMPH gene, UNC5B gene, OAT gen, EZR gene or NR4A2 gene, and
   ▪ the biological sample isolated from the subject is blood or adipose tissue; and
(ii) comparing the expression levels obtained after the therapy with the expression levels of said gene in a control sample, wherein
   - If the expression levels of SLC18A2 gene after the therapy are lower than the control sample, then response of the subject to the therapy is positive; or
   - If the expression levels of RIMS1 gene after the therapy are higher than the control sample, then response of the subject to the therapy is negative; or
   - If the expression levels of NUDT2 gene after the therapy are similar to the ones in the control sample, then response of the subject to the therapy is positive; or
   - If the expression levels of AMPH gene are similar to the ones in the control sample, then response of the subject to the therapy is positive; or
   - If the expression levels of UNC5B gene are similar to the ones in the control sample, then response of the subject to the therapy is positive; or
   - If the expression levels of OAT gene are similar to the ones in the control sample, then response of the subject to the therapy is positive; or
   - If the expression levels of EZR gene are similar to the ones in the control sample, then response of the subject to the therapy is positive; or
   - If the expression levels of NR4A2 gene are similar to the ones in the control sample, then response of the subject to the therapy is positive.

In the context of the present invention, the expression "monitoring the response to a therapy" refers to assess if the compounds directed to reduce to cognitive impairment are being effective or not. It is considered that the response of the subject to a therapy is positive or effective, if the therapy administered to the subject reduces the cognitive impairment. On the contrary, it is considered that the response of the subject to a therapy is negative or not effective, if the therapy administered to the subject does not reduce the cognitive impairment of the subject. Methods for measuring the cognitive impairment increase or decrease are widely known in the state of the art.

The terms "obesity/overweigh", "cognitive impairment", "subject", "control sample" etc. have been defined previously to the diagnosis method of the invention, being applicable to the monitoring method of the invention.

In the context of the monitoring method of the invention, "the expression levels of a gene are similar to the ones in the control sample" means that there is not statistically significative difference between both expression levels.

In a particular embodiment of the "monitoring method of the invention", the cognitive impairment is Mild Cognitive Impairment (MCI), Age-related Cognitive Decline (ARCD) or Age-Associated Memory Impairment (AAMI).

In another particular embodiment of the "monitoring method of the invention", alone or in combination with all or each one of the previous particular embodiments, the subject is a subject with obesity or overweight, preferably, the subject is a human subject having a body Mass Index (BMI) equal or higher than 25 kg/m², preferably, equal or higher than 30 Kg/m², more preferably, equal or higher than 35 Kg/m², still more preferably, equal or higher than 40 Kg/m².

In another particular embodiment of the "monitoring method of the invention", determining the expression levels of the gene comprises measuring the level of cDNA, mRNA and/or the protein encoded by said gene. Methods for determining the expression levels of a gene have been explained in previous paragraphs.

To implement the present invention, it is necessary to have a kit comprising the reagents needed to determine the expression levels of a gene specifically expressed in adipose tissue. Therefore, in another aspect, the present invention relates to the use of a kit (i) for the *in vitro* diagnosis of a cognitive impairment in a subject, or (ii) for monitoring the response of a subject to a therapy directed to improve the cognitive function in a subject suffering from cognitive impairment, hereinafter "use of the kit of the invention", wherein
- the kit comprises the reagents needed to determine the expression levels of one or more genes specifically expressed in adipose tissue, or of a fragment of said genes, and
- said genes are selected from the list consisting of the SLC18A2 gene, RIMS1 gene, NUDT2 gene, AMPH gene, UNC5B gene, OAT gen, EZR gene, NR4A2 gene, and combinations thereof.

In the context of the present invention, kit is understood as the product containing the reagents needed to carry out the method of the invention adapted to enable the transport and storage thereof. The materials that are suitable for packaging the components of the kit include, but are not limited to, polyethylene, polypropylene, polycarbonate and similar, glass, plastic, etc. The kit can further comprise bottles, jars, paper, envelopes, etc.

The expression "reagents needed to determine the expression levels of a gene" means a compound or a group of compounds that enables the expression level of a gene to be determined, both by means of determining the cDNA or mRNA level and by means of the protein level. Therefore, the reagents of the first type include nucleic acids, e.g. primers and probes, capable of specifically hybridising with the mRNA encoded by the gene involved. The reagents of the second type are compounds that specifically bind to the protein encoded by the gene and, preferably, include the antibodies although they can be specific aptamers.

In a particular embodiment of the use of the kit of the invention, alone or in combination with all or each one of the previous particular embodiments, the subject is a subject with obesity or overweight, preferably, the subject is a human subject having a body Mass Index (BMI) equal or higher than 25 kg/m², preferably, equal or higher than 30 Kg/m², more preferably, equal or higher than 35 Kg/m², still more preferably, equal or higher than 40 Kg/m².

In another particular embodiment of the use of the kit of the invention, the reagents needed to determine the expression levels of the genes are
- a nucleic acid that specifically hybridises the gene, or with a fragment thereof, or
- antibodies that specifically recognise the protein encoded by the gene, or that specifically recognise a fragment of said protein.

An example of a nucleic acid that specifically hybridises with gene is, for example, a probe or a primer. As used herein, the term "primer" refers to a single-stranded polynucleotide that is capable of hybridizing to a nucleic acid and allowing polymerization of a complementary nucleic acid, generally by providing a free 3'-OH group. In the present invention, "probe" is understood as a nucleic acid molecule whose nucleotide sequence specifically hybridises with the nucleotide sequence of a target gene. In the present invention, the target gene is a gen specifically expressed in adipose tissue, or of a fragment of said genes, such as the SLC18A2 gene, RIMS1 gene, NUDT2 gene, AMPH gene, UNC5B gene, OAT gen, EZR gene, or NR4A2 gene. The expression "specifically hybridises", as herein used, refers to the conditions that enable the hybridisation of two polynucleotides in highly or moderately rigorous conditions. Highly rigorous conditions generally imply: (1) low ionic strength and high temperature for washing, for examples, 0.015M sodium chloride/ 0.0015M sodium citrate/0.1% sodium dodecyl sulphate at 50°C, (2) use during the hybridisation of a denaturing agent, such as formamide, for example, 50% (v/v) formamide with 0.1% of bovine serum albumin/0.1% Ficoll/0.1% polyvinylpyrrolidone/50 mM sodium phosphate buffer at pH 6.5 with sodium chloride at 750 mM, 75 mM sodium citrate at 42ºC, or (3) use of 50% formamide, 5xSSC (0.75 M NaCl, 0.075 M sodium citrate), 50mM sodium phosphate (pH 6.8), 0.1% sodium pyrophosphate, 5 times Denhardt's solution, DNA of sodium salt from salmon testes (50 mg/mL), 0.1% SDS, and 10% dextran sulphate at 42ºC, with washes at 42ºC in 0.2xSSC (sodium chloride/sodium citrate) and 50% formamide, followed by highly rigorous washing consisting of 0.1xSSC that contains EDTA at 55ºC. "Moderately rigorous conditions" include the use of washing solution and hybridisation conditions that are less strict than those described above. How to carry out the hybridisation of two nucleotide sequences can be found is routine practise to the skilled person in the art.

Thus, in a more particular embodiment of the use of the kit of the invention, the nucleic acid that specifically hybridises the gene is primers or probes.

In an even more particular embodiment of the use of the kit of the invention, the primers are SEQ ID NO: 17 to SEQ ID NO: 30.

In the case that the expression levels of gene/s are determined by measuring the levels of the protein encoded by said gene, the kit comprises reagents that are capable of specifically binding to said protein, such as antibodies. Examples of antibodies that can specifically recognise the protein encoded by a gen specifically expressed in adipose tissue, or of a fragment of said genes, such as the SLC18A2 gene, RIMS1 gene, NUDT2 gene, AMPH gene, UNC5B gene, OAT gen, EZR gene, or NR4A2 gene, and bind to it have been described in preceding paragraphs.

In another aspect, the present invention relates to a composition, preferably, a pharmaceutical composition, comprising, or consisting of, a modulating agent of the adipose tissue gene expression levels for use in the treatment of a cognitive impairment in a subject, wherein the adipose tissue gene is SLC18A2 gene, RIMS1 gene, NUDT2 gene, AMPH gene, UNC5B gene, OAT gen, EZR gene or NR4A2 gene. Hereinafter "use of the composition of the invention".

The terms "cognitive impairment", "subject, "obesity", "overweight", etc. have been defined previously in the present description.

As used herein, the term "treating" (or "treat" or "treatment") refers to fighting the effects caused as a consequence of the disease or pathological condition of interest (like tissue damage) in a subject (preferably a mammal and, more preferably, a human), including:
i) inhibiting the disease or pathological condition, i.e., stopping its development;
(ii) alleviating the disease or pathological condition, i.e. causing the regression of the disease or pathological condition or its symptoms;
(iii) stabilizing the disease or pathological state.

In the present invention, the condition or disorder to be treated is cognitive impairment in a subject. In a particular embodiment of the use of the composition of the invention, the subject is a subject having obesity or overweight, preferably, the subject is a human subject having a body Mass Index (BMI) equal or higher than 25 kg/m², preferably, equal or higher than 30 Kg/m², more preferably, equal or higher than 35 Kg/m², still more preferably, equal or higher than 40 Kg/m².

In another particular embodiment of the use of the composition of the invention, the cognitive impairment is Mild Cognitive Impairment (MCI), Age-related Cognitive Decline (ARCD) or Age-Associated Memory Impairment (AAMI).

As use herein, the term "modulating" refers to (i) the up-regulation of, or otherwise increasing, the expression of one or more genes, to (ii) the down-regulation of, or otherwise decreasing, the expression of one or more genes, to (iii) inhibiting or otherwise decreasing the expression, activity and/or function of one or more gene products, and/or (iv) enhancing or otherwise increasing the expression, activity and/or function of one or more gene products. In the context of the present invention, the target gene is a gen specifically expressed in adipose tissue, preferably, the adipose tissue gene is SLC18A2 gene, RIMS1 gene, NUDT2 gene, AMPH gene, UNC5B gene, OAT gen, EZR gene or NR4A2 gene. In order to treat the cognitive impairment in a subject according to the present invention, the expression of the SLC18A2 gene, or the function of the protein encoded by it, has to be down-regulated; and/or the expression of the RIMS1 gene, NUDT2 gene, AMPH gene, UNC5B gene, OAT gen, EZR gene or NR4A2 gene has to be up-regulated.

Examples of modulating agents to up-regulate or to down-regulate the expression levels of a gene/protein include, without limiting to, (i) an antibody or fragment thereof; (ii) a small molecule; and (iiii) a siRNA, an antisense oligonucleotide, a nuclease or a ribozyme. The skilled person in art, knowing the nucleotide/amino acid sequence of a genes/protein, can identify and prepare modulating agents specific for the genes/proteins cited, being capable of up-regulating or of down-regulating the expression levels of a gene.

Examples of a modulating agent to up-regulate the expression levels of a gene include, without limiting to, miRNA.

Thus, in another particular embodiment of the use of the composition of the invention, the modulating agent of the gene expression levels is a miRNA or an iRNA that specifically hybridises the gene, or an antibody that specifically recognises the protein encoded by the gene.

According to the conventional techniques known to those skilled in the art, the composition according to the present invention may be formulated in combination with an excipient and/or a carrier. Thus, in a particular embodiment, the composition of the invention further comprises an excipient and/or a carrier.

The term "excipient" refers to a substance which helps to absorb any of the components of the composition of the invention, stabilizes said components or helps in the preparation of the composition in the sense of giving it consistency or providing flavours which make them more pleasant. Thus, excipients could have the function of keeping the components bound together, such as for example starches, sugars or celluloses, a sweetening function, a colorant function, the function of protecting the active ingredient, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material which, included in the galenic forms, is added to the active ingredients or to its associations to enable its preparation and stability, modify its organoleptic properties or determine the physical/chemical properties of the composition and its bioavailability. The "excipient" should allow for the activity of the compounds of the composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerine, amongst others.

The term "carrier" is preferably an inert substance. The function of the carrier is to facilitate the incorporation of other compounds, to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency and form to the composition.

In another particular embodiment, the composition of the invention is a pharmaceutical composition. In this case, the excipient or the carrier of the composition is a "pharmaceutically acceptable excipient" or a "pharmaceutically acceptable carrier". This means that the carrier or the excipient should allow for the activity of the compounds of the pharmaceutical composition, that is to say, for it to be compatible with said components. When the form of presentation is liquid, the pharmaceutically acceptable carrier is the diluent. Likewise, a composition, including its components, is said to be "pharmacologically acceptable" if its administration can be tolerated by a recipient subject, such as a mammal.

The pharmaceutical composition of the invention for its use in the treatment of cognitive impairment in a subject having obesity or overweight may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions and the like, containing the compounds of the present invention are employed. Similarly, transdermal patches may also be used for topical administration.

### DESCRIPTION OF THE DRAWINGS

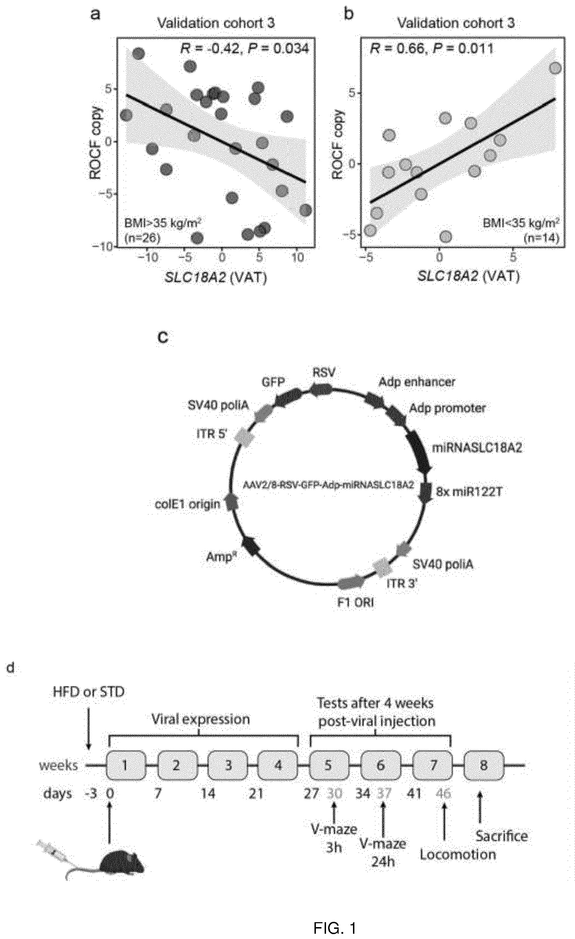

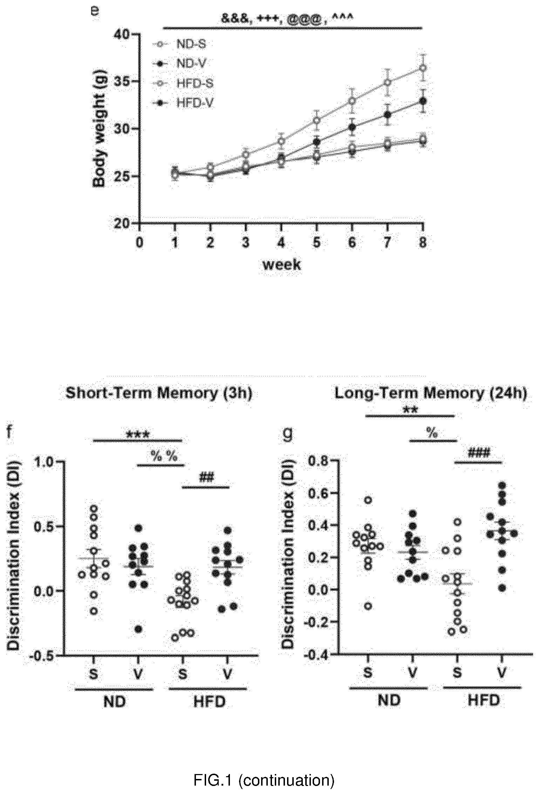

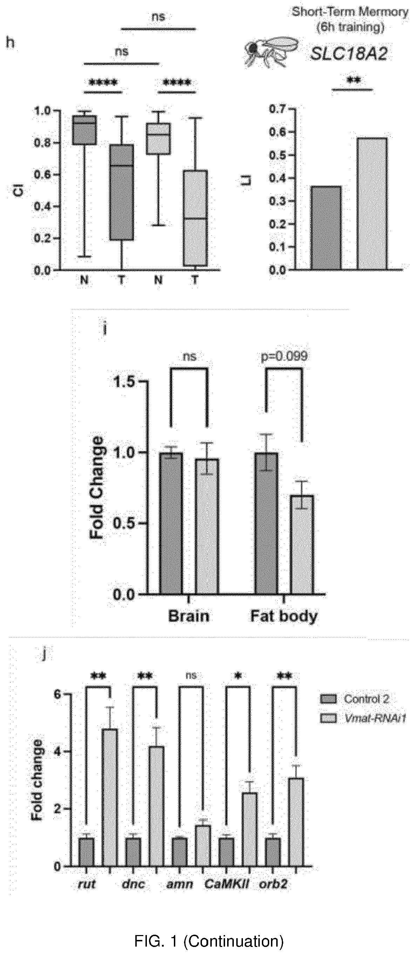

**Figure 1****.** Associations of *SLC18A2* with cognition in the validation cohort 3 and altered expression in pre-clinical models. Scatter plots of the partial Spearman's correlations (adjusted for age, BMI, sex, education years) between the SLC18A2 VAT expression and the Rey-Osterrieth complex figure (ROCF) copy scores in a) morbid obese patients (BMI>35 kg/m2) and b) patients with a BMI<35 kg/m2 (Adipobrain cohort, n=40). c) Schematic representation of the AAV packaging plasmid used to downregulate SLC18A2 gene expression, d) Timeline of events and mice cognitive testing. Arrows indicate punctual events such as virus injection, tests and sacrifice. e) Weekly body weight (g). Mean ± SEM; n=12 normal diet + saline (ND-S), n=11 normal diet + virus (ND-V), n=13 high fat diet + saline (HFD-S), n=12 high fat diet + virus (HFD-V). &&& P < 0.001 week effect; + P < 0.05, +++ P < 0.001 diet effect; $ P < 0.05 treatment effect; @@@ P < 0.001 week x diet interaction; ^^^ P < 0.001 week x treatment interaction (three-way ANOVA). f-g) Short- (3h) and long-term (24h) memory using the novel object recognition test. Dots with the mean ± SEM. P < 0.01, *** P < 0.001 ND-S vs HFD-S; %% P < 0.01 ND-V vs HFD-S; ##P < 0.01 HFD-S vs HFD-V (two-way ANOVA). h) Male short-term memory in the courtship conditioning paradigm. Control 2 (w; C7-GAL4/+; UAS-Dcr-2/+) and Vmat-RNAi1 fat body-specific knockdown flies (w; C7-GAL4/+; UAS-Dcr-2/ Vmat -RNAi1). i) Relative gene expression of Vmat in fly heads and fat body, and j) rutabaga (rut), dunce (dnc), amnesiac (amn), homer, CAMKII, and orb2 in fly heads of Vmat-RNAi1 fat body-specific knockdown flies and its corresponding genetic background control. Mean ± SEM. (t-test: * P<0.05, ** P <0.01, **** P <0.0001). Data based on a minimum of five biological and two technical replicates. Panels c and d were created with BioRender.com.

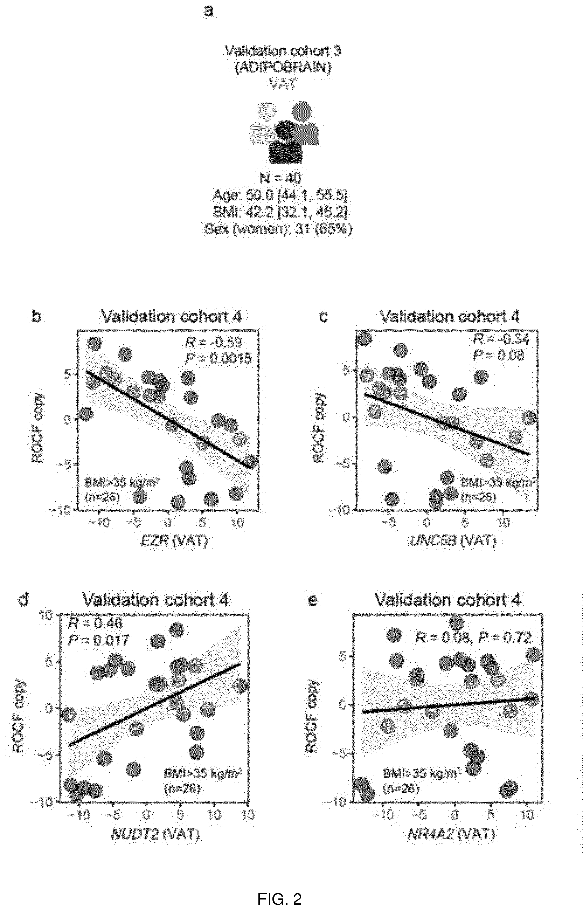

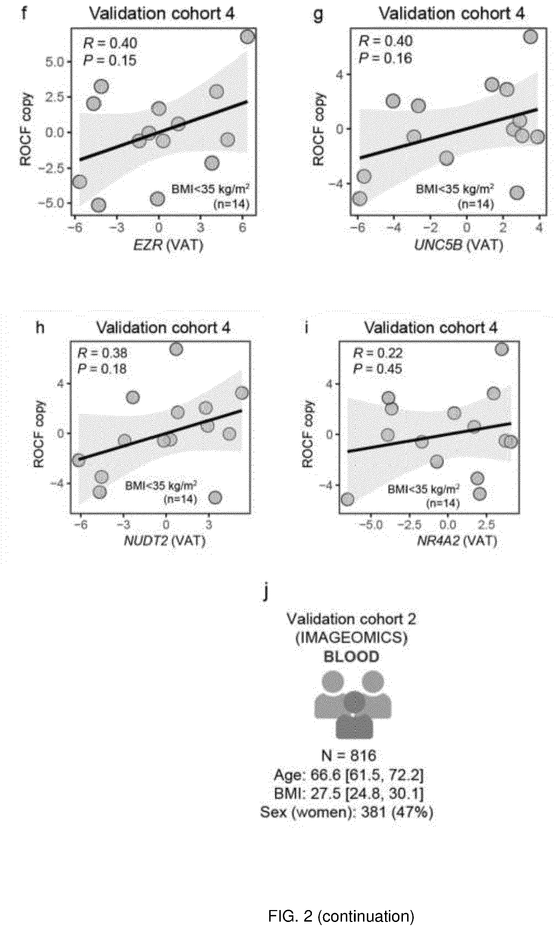

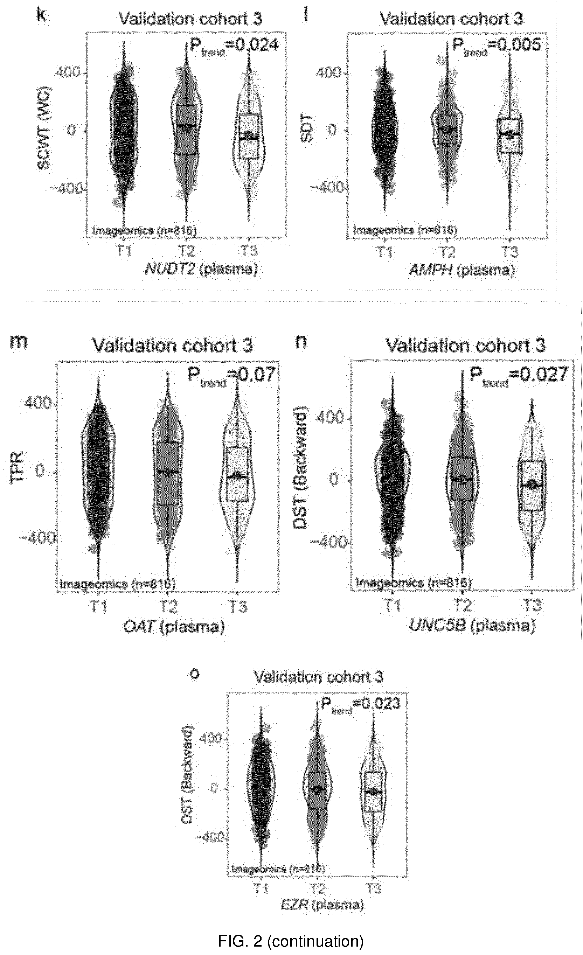

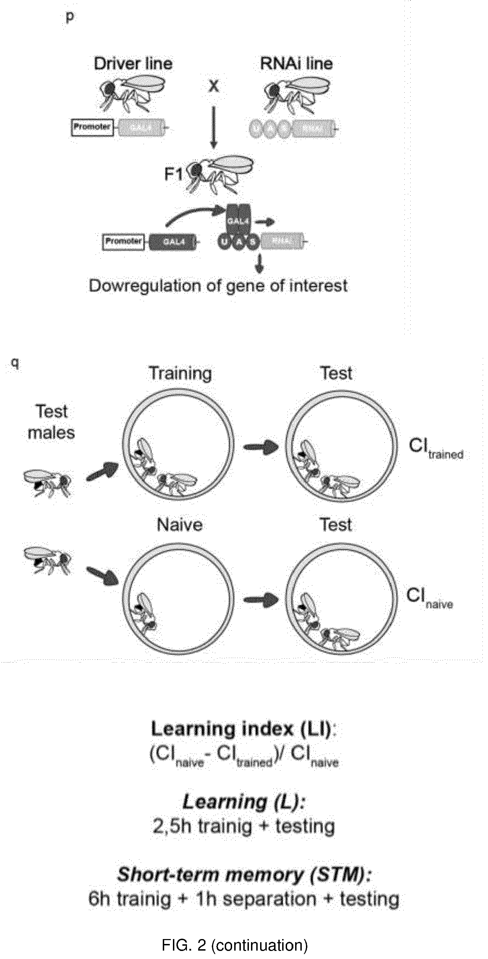

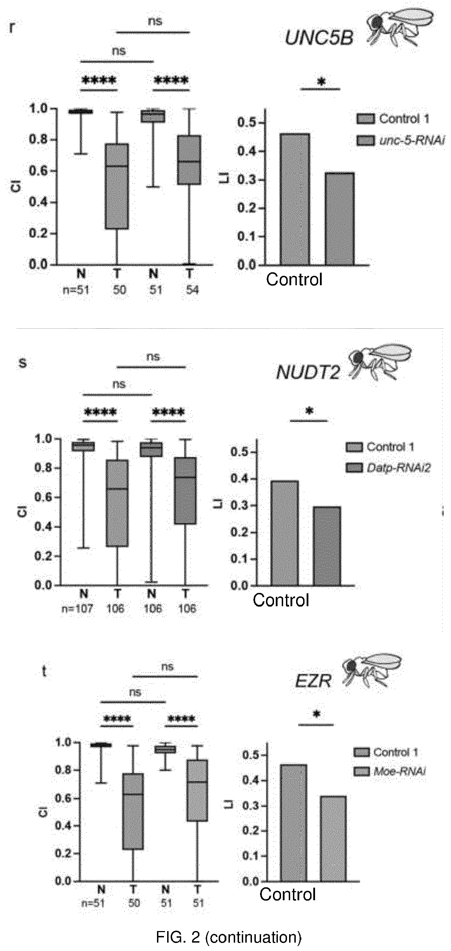

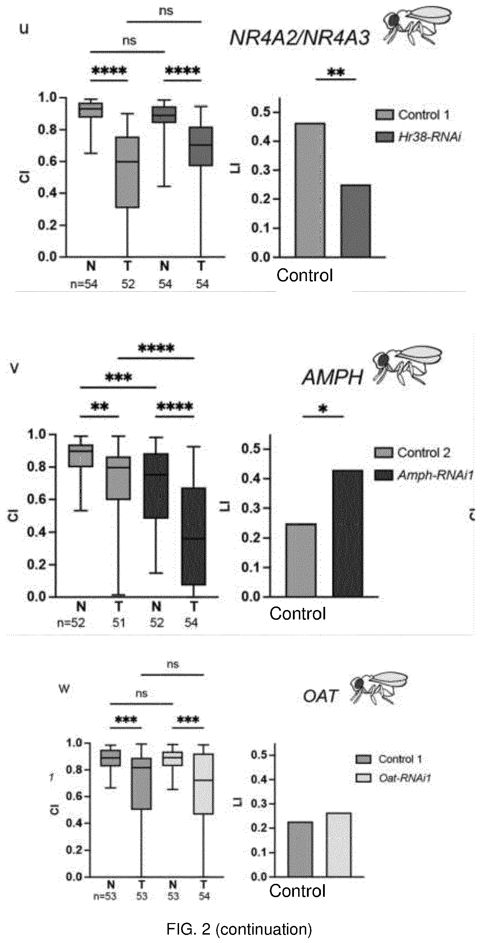

**Figure 2****.** Associations of expression levels of selected genes with cognition in a validation cohort, in the Aging Imageomics cohort and *Drosophila melanogaster.* a) Main baseline characteristics of the validation cohort 3 (Adipobrain). Scatter plots of the partial Spearman's rank correlations (adjusted for age, BMI, sex, and education years) between Rey-Osterrieth complex figure (ROCF) copy scores and the VAT expression levels of b) *EZR, c*) *UNC5B*, d) *NUDT2,* and e) *NR4A2* in patients with a BMI>35 kg/m² from the Adipobrain cohort. f-i) The same associations but in patients with a BMI<35 kg/m². j) Main baseline characteristics of the validation cohort 2 (Imageomics). Violin plots of the score in several cognitive tests and the tertiles (T1, T2, T3) of the circulating expression levels of selected genes: k) Normalized Stroop Colour Word Test-Colour Word (SCWT CW) *vs NUDT2;* /) Symbol Digit Test (SDT) *vs AMPH;* m) Total Paired Recall (TFP) *vs OAT,* n) Normalized Digit Span Test (DST) Backward *vs UNC5B;* o) Normalized DST Backward *vs EZR.* The ranked residuals after controlling for age, BMI, gender and education level are plotted. Overall significance was assessed using a Mann-Kendall trend test. p) Scheme of the RNA interference via the UAS-GAL4 system. q) Courtship conditioning paradigm. r-w) The graphs display courtship conditioning paradigm results of fat body promoter line *w; C7-GAL4; UAS-Dcr-2* crossed with RNAi lines targeting r) *unc-5,* s) *Datp,* t) *Moe,* u) *Hr38,* v) *Amph,* w) *Oat* and their corresponding genetic background controls (Control 1 and 2). Box plots represent the Cl of naive (N) and trained (T) males. Significance of courtship suppression upon training was assessed with Kruskal Wallis non-parametrical test and post hoc Dunn's multiple comparison test. LI statistical significance was determined with the non-parametrical bootstrap analysis with 10000 iterations. *p < 0.05, **p < 0.01, ***p < 0.001.

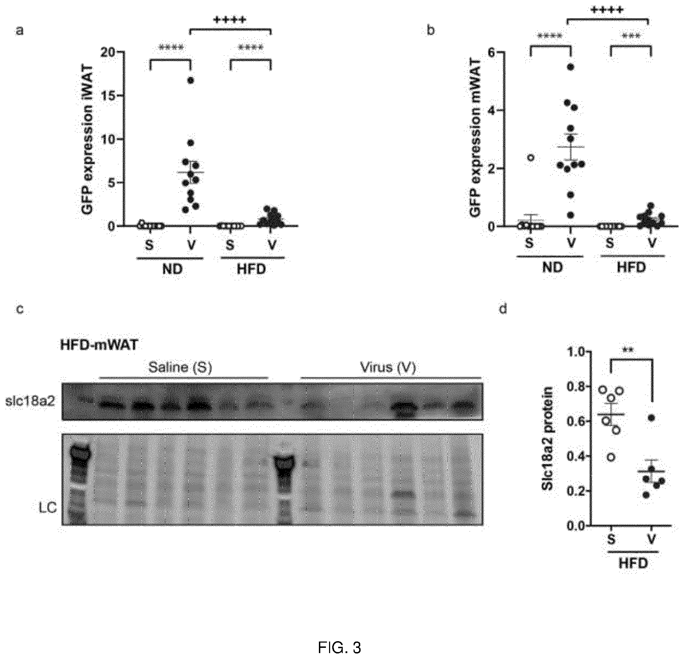

**Figure 3****.** The expression levels of green fluorescent protein (GFP) contained in the vector as a reporter gene was significantly increased in both inguinal and mesenteric white adipose tissue (iWAT and mWAT) of mice from the vectors-injected groups compared to the saline groups (Figures 3a, 3b), thereby indicating that the vector was able to reach the adipose tissue. We also found a significant decrease in the slc18a2 protein levels in the mWAT of virus-injected mice fed a HFD compared to their respective controls (Figure 3c, 3d).

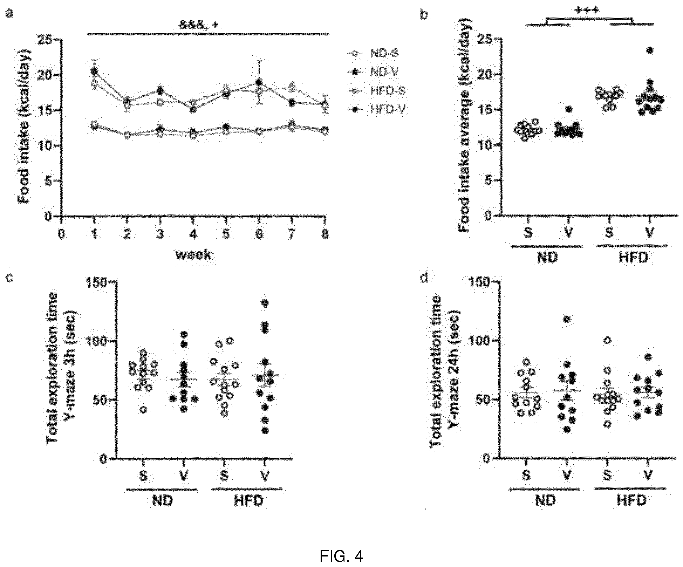

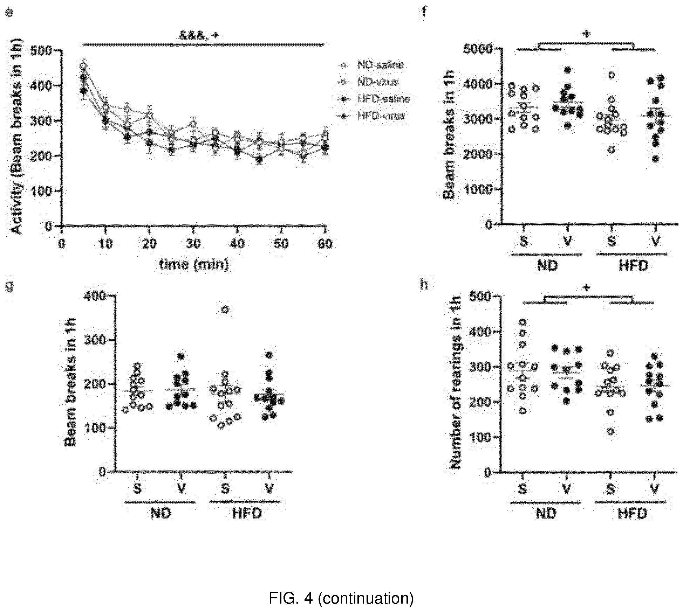

**Figure 4****.** Mice fed a HFD gained more weight in parallel to food intake compared with those fed a ND (Figure 1e, Figure 4a, 4b). Mice fed a HFD had lower short- and long-term memory than mice fed a ND (Figure 1f, 1g). Downregulation of slc18a2 in the mice adipose tissue reversed the memory impairment induced by a HFD (Figure 1f, 1g). No differences were observed in the exploration time between groups (Figure 4c, 4d). Although a diet effect on locomotion, downregulation of slc18a2 had no significant effect (Figures 4e-4h).

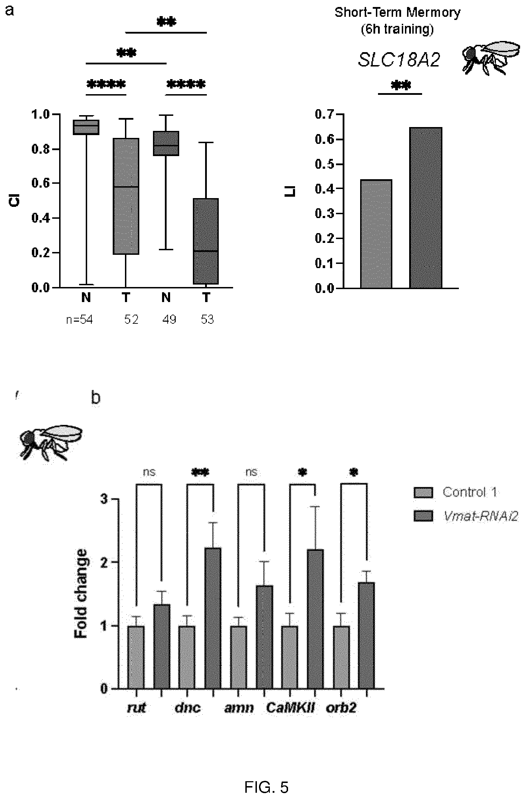

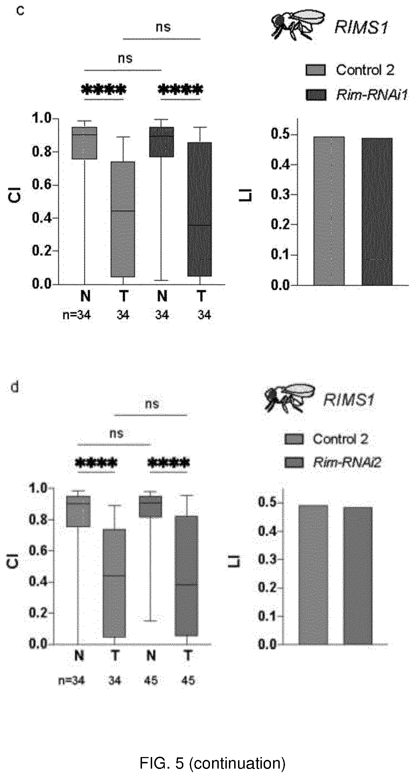

**Figure 5****.** Data showing the Up-regulation and Down-regulation of genes.

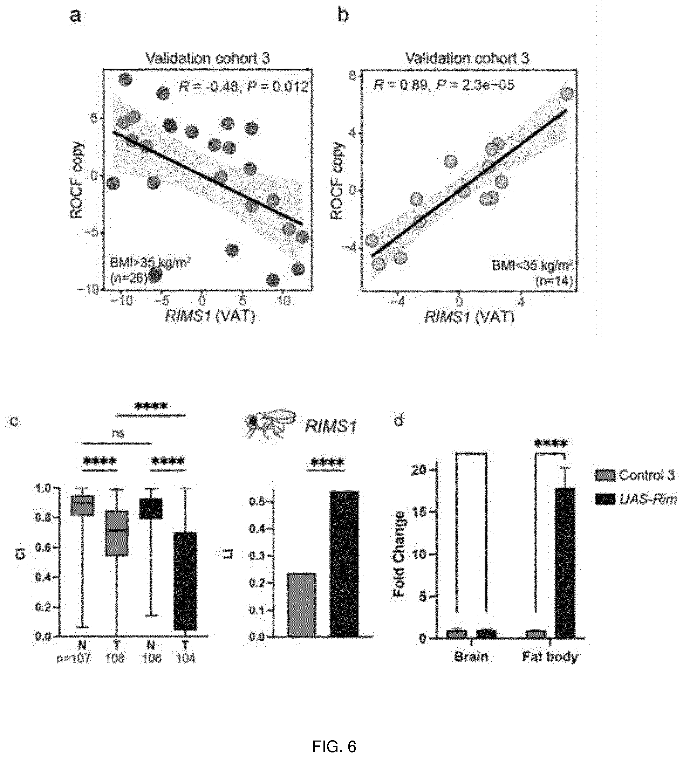

**Figure 6**. Figure 7. Associations of ***RIMS1* with cognition in the validation cohort 3 and Rim overexpression in *Drosophila* fat body effects in learning and gene expression profiles.** a, b) Scatter plots of the partial Spearman's rank correlations (adjusted for age, BMI, sex, and education years) between the VAT expression levels of *RIMS1* and the ROCF copy scores in a) morbid obese patients (BMI>35 kg/m²) and b) patients with a BMI<35 kg/m² from the Adipobrain cohort (n=40). c) *Rim* overexpression in the *Drosophila* fat body and associations with learning. differences in courtship index (CI) between naive (N) and trained (T) males were assessed with Kruskal Wallis non parametrical test and post hoc Dunn's multiple comparison test. Learning indexes (LI) to asses either short-term memory (for *Vmat*) or learning (for *Rim*) were calculated from Cls as specified in the material and methods section. Statistical significance was determined with the non-parametrical bootstrap analysis with 10000 replicates. * *P* < 0.05, ** *P* < 0.01, *** *P* < 0.001, **** *P* < 0.0001. d) *Rim* relative expression assessed by qRT-PCR in fly brain or fat body in *UAS-Rim* fat body-specific overexpression flies and its corresponding genetic background control. Data are derived from a minimum of five biological and two technical replicates. e) Dot plot of significantly (qvalue<0.1) over-represented Reactome pathways, f) gene ontology biological processes (GO-BP), and g) molecular functions (GO-MF) associated to the miss expressed genes in the fat body of *UAS-Rim* flies. h) Gene-concept network depicting significant genes involved in enriched Reactome pathways (from panel e) and b) GO-BP (from panel f). j) Over-representation analysis of pathways (qvalue>0,1) associated with differentially expressed gene transcript in *UAS-Rim* fly heads based on the Reactome database. k) Gene-concept network associated to significant genes involved in Reactome pathways of panel j. I) Significantly over-represented gene ontology molecular functions of significant gene transcripts in fly heads (qvalue>0,1).

### EXAMPLE

### I- MATERIAL AND METHODS

### Clinical cohorts

*Discovery cohort* (*IRONMET,* n=17). From January 2016 to October 2017, a cross-sectional case-control study was undertaken in the Endocrinology Department of Josep Trueta University Hospital (Girona, Spain). In 17 morbidly obese patients (BMI > 35 mg/kg²) aged 28-60 years old, adipose tissue stranded RNA-seq was analysed.

*Validation cohort* 1 (*INTESTINE,* n=22) . Consecutively, 22 morbidly obese (BMI>35 kg/m²) subjects with different degrees of insulin action (measured using hyperinsulinemic-euglycaemic clamp) were recruited at the Endocrinology Service of the Hospital Dr Josep Trueta to validate adipose tissue RNA-seq analysis.

In both cohorts, all subjects were of Caucasian origin and reported a body weight stable for at least three months before the study. Subjects were studied in the post-absorptive state. Exclusion criteria were: previous type 2 diabetes mellitus, chronic inflammatory systemic diseases, acute or chronic infections in the previous month; severe disorders of eating behaviour or major psychiatric antecedents; neurological diseases, history of trauma or injured brain, language disorders; and excessive alcohol intake (≥ 40 g OH/day in women or 80g OH/day in men). Body fat composition was estimated using Bio-electrical impedance analysis (BC-418, Tanita Corporation of America, Illinois, USA). This protocol was revised, validated and approved by the Ethics committee of the Hospital Dr. Josep Trueta. The purpose of the study was explained to participants and they signed written informed consent before being enrolled in the study.

*Validation cohort 2 (IMAGEOMICS,* n=816) . The Aging Imageomics Study is an observational study including participants from two independent cohort studies (MESGI50 and MARK). Detailed description of the cohorts can be found elsewhere . Briefly, the MESGI50 cohort included a population aged ≥ 50 years old, while the MARK cohort included a random sample of patients aged 35-74 years with intermediate cardiovascular risk. Eligibility criteria included age ≥ 50 years, dwelling in the community, no history of infection during the last 15 days, no contraindications for MRI, and consent to be informed of potential incidental findings. The Aging Imageomics Study protocol was approved by the ethics committee of the Dr. Josep Trueta University Hospital.

*Validation cohort 3 (ADIPOBRAIN,* n=40). All the samples and data of the participants included in this study were provided by the FATBANK (platform promoted by the CIBEROBN and coordinated by the IDIBGI Biobank), integrated in the Spanish National Biobank Network. A total of 40 patients ages 40 to 64 years were included. From these, 26 subjects had morbid obesity (BMI > 35 mg/kg²), aged between 40 and 63. Patients had no systemic disease other than obesity and all were free of any infection in the month prior to the study. Liver disease (specifically tumour disease and HCV infection) and thyroid dysfunction were specifically excluded. Adipose tissue samples were obtained from VAT depots during elective surgical procedures.

### Neuropsychological Assessment

The primary endpoint in all these cohorts was the study of cognitive function. In these subjects, the following tests were collected .

*Digit Span tests.* The Digit Span is a subtest of the Wechsler Adult Intelligence Scale-III (WAIS-III) (J. Puig, et al. 2020. Mech Ageing Dev. 189, 111257). It is based on numbers and includes the Forward and the Backward Digit Span tests. In the Forward Digit Span test, the examinee repeats a number sequence in the same order as presented. This constitutes a measure of working memory but also of attention. In the Backward Digit Span task, the examinee repeats in reverse order series of digits that became gradually longer. This is an executive task particularly dependent on working memory. A higher score reflects a better working memory. In a standardization sample of 394 participants (aged 16-89 years), the reliability coefficient was very high, ranging from 0.94-0.97 .

*Trail Making Test.* The Trail Making Test (TMT) is composed by two parts: part A (TMTA) and part B (TMTB). The TMTA (greater focus on attention) consisted of a standardized page in which numbers 1 to 25 are scattered within the circles, and participants were asked to connect the numbers in order as quickly as possible. Before starting the test, a 6-item practice test was administered to the participants to make sure they understood both tasks. A maximum time of 300 s was allowed before suspending the test. The direct scores of TMTA were the time in seconds taken to complete each task. In the same way, the TMTB (greater focus on executive function) consisted of an alternating sequence of numbered circles and letters J. D. Corrigan, N. S. Hinkeldey, 1987. J Clin Psychol. 43, 402-409; M. D. Lezak, 1984. Scand J Work Environ Health. 10, 25-29 . In both tests, shorter times to completion indicate better performance.

*California Verbal Learning Test-II (CVLT).* The CVLT is used to assess verbal learning and memory . It consists of 5 learning tests in which a list of words (list A) is presented and the subject is asked, immediately after each presentation, to recall as much words as possible. Then an interference list (list B) is presented, and the subject is asked to repeat the same task. CVLT Immediate Recall score is a result of the first five tests and provides information about the learning process. In the short delay test, the patient is asked to recall list A, free (CVLT Short Delayed Free Recall) or with semantic facilitation (CVLT Short Delayed Cued Recall). After 20 minutes, in which non-verbal tasks are carried on, the subjects are asked to repeat a list of words that the examinee has previously presented without semantic facilitation (The CVLT Long Delayed Free Recall) to assesses long-term memory. A higher score reflects a better memory function. About 50 min are necessary to administrate this test and its reliability ranges from 0.78-0.94.

*Stroop Test* (Golden's version). The Stroop test was used to assess cognitive flexibility, selective attention, inhibition and information processing speed. This version consists of three different parts: 1) 100 words (colour names) are printed in black ink and the subject is asked to read them as fast as possible, 2) 100 "XXX" are printed in Colour ink (green, blue and red) and the subject is asked to name as fast as possible the ink Colour, and 3) 100 Colour names (from the first page) printed in Colour ink (from the second page), the Colour name and the ink Colour do not match and the subject is asked to name the ink Colour (and not to read the Colour name). The subject is given 45 seconds for each task. After the 45 seconds the last item completed is noted, obtaining three scores: one for each part of the test ("W", "C" and "CW"). The interference ("I") index was also obtained from the subtraction CW-CW, where CW'= (C *×* W) / (C +W). Standard administration procedures were followed as indicated in the test manual.

*Memory Binding Test (MBT).* The MBT consists of 16 category cues two-word lists containing 16 word items in each list. Both lists are learned and recalled by controlled learning and cued and paired recall. The MBT produces four principal measures, the total paired recall (TPR), total free recall (TFR), total delayed free recall (TDFR) and total delayed paired recall (TDPR).

*Symbol Digit Test (SDT).* The SDT is a subtest of the Wechsler Adult Intelligence Scale-III (WAIS-III). It includes a coding key that shows nine abstract symbols, each paired with a number and below the key, series of symbols were presented. The participants were asked to write down the corresponding numbers associated with the abstract symbols as quickly as possible. This task is a measure of information processing speed. The number of correct substitutions during a 90-second interval was scored, and higher numbers indicate better performance.

### General Cognition

A composite cognitive score was computed as the sum of the standardized scores of each individual for each test included in the Aging Imageomics cohort.

### Adipose tissue collection and handling.

Adipose tissue samples were obtained from subcutaneous (SAT) and visceral (VAT) adipose tissue during elective surgical procedures (cholecystectomy, surgery of abdominal hernia and gastric bypass surgery). Both SAT and VAT samples were collected from the abdomen, following standard procedures. Samples of adipose tissue were immediately transported to the laboratory (5-10 min). The handling of tissue was carried out under strictly aseptic conditions. Adipose tissue samples were washed in PBS, cut off with forceps and scalpel into small pieces (100 mg), and immediately flash-frozen in liquid nitrogen before stored at -80ºC.

**VAT and SAT stranded RNA sequencing** *(Discovery IRONMET; validation cohort 1 INTESTINE).*
VAT and SAT RNA purification was performed using RNeasy-Tissue Mini-Kit (QIAgen). Total RNA was quantified by Qubit^{®} RNA BR Assay kit (Thermo Fisher Scientific) and the integrity was checked by using the RNA Kit (15NT) on 5300 Fragment Analyzer System (Agilent).

The RNA-seq libraries were prepared with Illumina^{®} TruSeq^{®} Stranded Total RNA Sample Preparation kit following the manufacturer's recommendations with some modifications. Briefly, in function of availability 100-500 ng of total RNA was ribosomal RNA (rRNA) depleted using the RiboZero Magnetic Gold Kit and fragmented by divalent cations. The strand specificity was achieved during the second strand synthesis performed in the presence of dUTP. The cDNA was adenylated and ligated to Illumina platform compatible IDT adaptors with unique dual indexes with unique molecular identifiers (Integrated DNA Technologies), for paired end sequencing. The ligation products were enriched with 15 PCR cycles and the final library was validated on an Agilent 2100 Bioanalyzer with the DNA 7500 assay (Agilent). The libraries were sequenced on NovaSeq 6000 (Illumina) in a fraction of sequencing flow cell with a read length of 2x101bp following the manufacturer's protocol for dual indexing. Image analysis, base calling and quality scoring of the run were processed using the manufacturer's software Real Time Analysis (RTA v3.4.4) and followed by generation of FASTQ sequence files.

RNA-seq reads were mapped against human reference genome (GRCh38) using STAR software version 2.5.3a (A. Dobin, et al. 2013. Bioinformatics. 29, 15-21**)** with ENCODE parameters. Genes were quantified using RSEM version 1.3.0 (B. Li, 2011. BMC Bioinformatics. 12 (2011), doi:10.1186/1471-2105-12-323*)* with default parameters and using the annotation file from GENCODE version 29. Only protein-coding genes that were expressed >1 cpm in at least 10 samples were considered for the association of gene expression with clinical variables.

**Circulating gene expression analysis** *(Validation cohort 2, Aging Imageomics).* Total RNA was purified from blood using PAXgene Blood RNA Kit (Qiagen, Gaithersburg, MD). RNA concentrations were assessed with Nanodrop ND-1000 Spectrophotometer (Thermo Fisher Scientific, Wilmington, DE). Total RNA was reversed transcribed to cDNA using HighCapacity cDNA Archive Kit (Applied Biosystems, Darmstadt, Germany). Gene expression was assessed by real time PCR using an LightCycler^{®} 480 Real-Time PCR System (Roche Diagnostics SL, Barcelona, Spain), using SYBR green technology suitable for relative genetic expression quantification. Glyceraldehyde-3-phosphate dehydrogenase (GAPDH) was used as endogenous control.

The commercially predesigned KiCqStart^{®} primers used were as follows:
- Nuclear receptor subfamily 4, group A, member 2 (NR4A2, forward sequence: 5'-GACTATCAAATGAGTGGAGATG-3' (SEQ ID NO: 17), reverse sequence: 5'-GACCTGTATGCTAATCGAAG-3' (SEQ ID NO: 18));
- Ornithine aminotransferase (OAT, forward sequence: 5'-CAGACCTGATATAGTCCTCC-3' (SEQ ID NO: 19), reverse sequence: 5'-CTTCTTCTA AAACCTCA AGGG-3' (SEQ ID NO: 20));
- Amphiphysin (AMPH, forward sequence: 5'-CCTGACA TAAAGAATCGCATC-3' (SEQ ID NO: 21), reverse sequence: 5'-CAAACACTT TCTGTGCTTTC-3' (SEQ ID NO: 22));
- Nudix hydrolase 2 (NUDT2, forward sequence: 5'-TTCTGCTT CGGTCCTTAG-3' (SEQ ID NO: 23), reverse sequence: 5'-AACTCAATTGCATTGTTGTC-3' (SEQ ID NO: 24));
- Unc-5 netrin receptor B (UNC5B, forward sequence: 5'-AGGACAGTTACCACAACC-3' (SEQ ID NO: 25), reverse sequence: 5'-GGGGATCTCCTGGTATTTG-3' (SEQ ID NO: 26));
- Ezrin (EZR, forward sequence: 5'-TGACTTTGTG TTTTATGCCC-3' (SEQ ID NO: 27), reverse sequence: 5'-CTCCTTTTCTT CTCTGTTTCC-3' (SEQ ID NO: 28)); and
- GAPDH (forward sequence: 5'-ACAGTTGCCATGTAGACC-3' (SEQ ID NO: 29), reverse sequence: 5'-TTGAGCACAGGGTACTTTA-3' (SEQ ID NO: 30)).

**VAT expression of candidate genes by RT-PCR** *(Validation cohort 3 ADIPOBRAIN*)*.* Total RNA was purified from VAT using RNeasy Lipid Tissue Mini Kit (QIAgen, Izasa SA). RNA concentrations were assessed with Nanodrop ND-1000 Spectrophotometer (Thermo Fisher Scientific, Wilmington, DE) and the integrity was checked by Agilent Bioanalyzer (Agilent Technologies, Palo Alto, CA). Total RNA was reversed transcribed to cDNA using High Capacity cDNA Archive Kit (Applied Biosystems, Darmstadt, Germany). Gene expression was assessed by real time PCR using a LightCycler^{®} 480 Real-Time PCR System (Roche Diagnostics SL, Barcelona, Spain), using TaqMan^{®} technology suitable for relative genetic expression quantification. Peptidylprolyl isomerase A (PPIA) and Glyceraldehyde-3-phosphate dehydrogenase (GAPDH) were used as endogenous control. The commercially available and pre-validated TaqMan^{®} primer/probe sets used were as follows: Solute carrier family 18 member A2 (SLC18A2, Hs00996835_m1); Regulating synaptic membrane exocytosis 1 (RIMS1, Hs01112189_m1), PPIA (Hs99999904_m1) and GAPDH (4352934). The commercially predesigned KiCqStart^{®} primers used were as follows: NUDT2, EZR, UNC5B, NR4A2 and PPIA.

**Plasma HPLC-ESI-MS/MS metabolomics** *(Discovery IRONMET).*
Metabolites were extracted from plasma samples with methanol (containing phenylalanine-C13 as an internal standard) according to previously described methods (J. Mayneris-Perxachs, et al. 2022. Cell Metab. 34, 681-701.e10; J. Mayneris-Perxachs, et al. 2022 Cell Host Microbe. 30, 340-356.e8; W. R. Wikoff, et al. 2008. Journal of Clinical Investigation. 118, 2661-9) . Briefly, samples 30µl of cold methanol were added to 10 µl of each sample, vortexed for 1 minute and incubated for one hour at -20 °C. Samples were homogenized using FastPrep-24^{™} (MP biomedicals) and were incubated overnight in a rocker at 4°C. Then, all samples were centrifuged for three minutes at 12.000g, the supernatant was recovered and filtered with a 0.2 µm Eppendorf filter. Two µL of the extracted sample were applied onto a reversed-phase column (Zorbax SB-Aq 1.8 µm 2.1 × 50 mm; Agilent Technologies) equipped with a precolumn (Zorbax-SB-C8 Rapid Resolution Cartridge 2.1 × 30 mm 3.5 µm; Agilent Technologies) with a column temperature of 60°C. The flow rate was 0.6 MI/min. Solvent A was composed of water containing 0.2% acetic acid and solvent B was composed of methanol 0.2% acetic acid. The gradient started at 2% B and increased to 98% B in 13 min and held at 98% B for 6 min. Post-time was established in 5 min.

Data were collected in positive and negative electrospray modes time of flight operated in full-scan mode at 50-3000 m/z in an extended dynamic range (2 GHz), using N2 as the nebulizer gas (5 L/min, 350°C). The capillary voltage was 3500 V with a scan rate of 1 scan/s. The ESI source used a separate nebulizer for the continuous, low-level (10 L/min) introduction of reference mass compounds 121.050873 and 922.009798, which were used for continuous, online mass calibration. MassHunter Data Analysis Software (Agilent Technologies, Barcelona, Spain) was used to collect the results, and MassHunter Qualitative Analysis Software (Agilent Technologies, Barcelona, Spain) to obtain the molecular features of the samples, representing different, co-migrating ionic species of a given molecular entity using the Molecular Feature Extractor algorithm (Agilent Technologies, Barcelona, Spain). We selected samples with a minimum of 2 ions. Multiple charge states were forbidden. Compounds from different samples were aligned using a retention time window of 0.1% ± 0.25 minutes and a mass window of 20.0 ppm ±2.0 mDa. We selected only those present in at least 50% of the samples of one group and corrected for individual bias.

**Plasma ¹H-NMR Metabolomics** *(Discovery IRONMET).* Plasma samples were thawed at room temperature. For each sample, 400 µL of plasma were combined with 200 µL of phosphate buffer ((9%w/V NaCl, 100% D₂O) that contained 10mM of 3-trimethylsilyl-1-[2,2,3,3-²H4] (TSP) (J. Mayneris-Perxachs, et al. 2022. Cell Metab. 34, 681-701.e10; J. Mayneris-Perxachs, et al. 2022 Cell Host Microbe. 30, 340-356.e8). Samples were mixed with the use of a vortex and centrifuged (10.000 × g) for 10 min. Then, a 550 µL aliquot was transferred into a 5 mm NMR tube prior to NMR analysis. ¹H spectra of low molecular weight metabolites were performed using a CPMG sequence (RD-90º-[t-180º- t]ₙ-ACQ-FID) with spin-echo delay of 400 µs (for a total T2 filter of 210 ms) allowing an efficient attenuation of the lipid NMR signals. The CPMG sequence generates spectra edited by T2 relaxation times, reducing broad resonances from high molecular weight compounds facilitating the observation of low molecular weight metabolites. The total acquisition time was 2.73 s with a RD of 2s and the 90º pulse length was automatically calibrated for each sample at around 11.1 µs. For each sample, 8 dummy scans were followed by 256 scans and collected in 64-K points over a spectral width of 20 ppm. TSP was used a general reference for NMR samples because it does not introduce any additional signals apart from the sharp methylsilyl resonance at 0 ppm. In addition, a high concentration of TSP was used to release low-molecular weight metabolites with high affinity for serum proteins by binding competition with TSP. All ¹H-NMR spectra were recorded at 300 K on an Avance III 600 spectrometer (Bruker^{®}, Germany) operating at a proton frequency of 600.20 MHz using a 5 mm PABBO gradient probe and automatic sample changer with a cooling rack at 4ºC.

**Plasma cytokines and neurotoxic proteins.** Commercially available ELISA kits were used to measure the plasma of IL-6 (RND-HS600C, Human IL-6 Quantikine HS ELISA Kit, R&D Systems), IL-4 ( RND-HS400, Human IL-4 Quantikine HS ELISA Kit, R&D Systems), alpha-synuclein (RND-DY1338-05, Human alpha-synuclein DuoSet ELISA, R&D Systems), amyloid b-42 (RND-DAB142, Human Amyloid beta (aa1-42) Quantikine ELISA Kit, R&D Systems), and tau (NBP2-62749, Human Tau ELISA Kit, Novus BiologicalsTM) following manufacturer's protocol.

### Drosophila melanogaster experiments

*Drosophila melanogaster stocks and maintenance.* Flies were raised on standard medium (cornmeal, sugar, yeast) and cultured according to standard procedures at 28°C in a 12:12 hours light dark cycle.
v60000 (Control I) and v60100 (Control 2) were obtained from Vienna *Drosophila* RNAi Centre (VDRC; http://stockcenter.vdrc.at/control/main) and used as the corresponding genetic background controls for KK and GD RNAi line collections, respectively. The *Rim* overexpression line (UAS-Rim), stock BL78050, referred to as *UAS-Rim* and the corresponding genetic background control (BL36304; Control 3) were obtained from the Bloomington *Drosophila* Stock Centre (BDSC; http://flystocks.bio.indiana.edu/). *UAS-Rim* was subsequently isomerized for 6 generations into the (BL36304; Control 3) genetic background. RNAi expression in the fat body was induced by *w;* C7-GAL4; *UAS-Dcr-2* driver line, which carry a fat tissue-specific promoter, driving the expression of GAL4 specifically on *Drosophila* adiposities, was kindly provided by Marek Jindra (J. Ryneset al. 2012. Mol Cell Biol. 32, 3949-3962).

*Drosophila courtship conditioning.* It was used to assess *Drosophila* learning and memory capabilities. Courtship conditioning was performed as described previously (T. S. Koemans, et al. 2017. Journal of Visualized Experiments. 2017, 55808). RNAi and overexpression lines together with their corresponding genetic background controls were crossed with *w;* C7-GAL4; *UAS-Dcr-2* driver line and raised at 28ºC to downregulate or overexpressed the corresponding targeted genes in the adipose tissue. Males were collected at eclosion and kept in isolation until 7 days of age, at 25ºC. Males were randomly assigned to either trained or naive groups. Learning capabilities were assessed by pairing individual males with a single pre-mated wild type female for 2.5 h during the training period. Immediately after training, the males were tested to assess Learning. Shorth-term memory was assessed by pairing individual males with a single pre-mated wild type female for 6 h during the training period and subsequently the male was individually isolated for 1 h previous to testing. Testing was performed by transferring male to a 1 cm diameter chamber with in the presence of a new pre-mated female and filmed for 8 min. The courtship behaviour of male flies towards females was quantified manually from videos. The mean courtship indexes (Cl, defined as the percentage of time spent on courtship during an 8-min interval) of trained males and of naive males were used to calculate the learning index (LI), which is defined as the per cent reduction in mean courtship activity in trained males compared with naive males; LI = (CIₙₐᵢᵥₑ-CI_{trained})/CIₙₐᵢᵥₑ. For all conditions, male-female pairs were analysed on a minimum of three different test days, and the data were pooled.

Non parametrical statistical bootstrapping analysis was preformed to assess differences in LI (Learning and short-term memory) between RNAi knockdown or overexpression lines and their corresponding genetic background controls. The R script provided by Koemans *et al.* (T. S. Koemans, et al. 2017. Journal of Visualized Experiments. 2017, 55808) was used to perform the bootstrapping analysis. Briefly, CI values were randomly sampled with replacement to generate 10000 hypothetical LIs, which were used to determine the 95% confidence interval of the difference between the index of the control and the index of the knockdown and the probability that this difference is >0. t test or one-way ANOVA were performed to assess statistical differences in courtship index (CI) of the compared conditions.

*mRNA extraction and cDNA synthesis.* Seven-day old male were selected for qRT-PCR or RNAseq. A total of ten adult heads or ten fat bodies were collected per sample and transferred to RNAlater solution (Sigma). Total RNA was extracted using the Arcturus PicoPure RNA Purification kit (Thermo Fisher Scientific). To avoid amplification from genomic DNA, DNase treatment was performed using the DNA-free Ambion kit and RNA was reverse transcribed into cDNA using the High-Capacity cDNA reverse transcription (Applied Biosystems) according to manufacturers' procedures.

*Quantitative real-time PCR (qRT-PCR).* Quantitative PCRs (qPCRs) were performed using the LightCycler 480 SYBR Green Master (Roche) in a LightCycler 480 II machine (Roche). Primer sequences are provided in and the reference genes RNApol2 and 1tub23cf are provided in Table 1.

**Table 1. Primer sequences for the Drosophila qRT-PCR analyses**

| SEQ ID NO: | PCR amplicon | Oligonucleotides (5' to 3') |
|---|---|---|
| 35 | Rim Fw | CAGCAACAGCCCAGATCAG |
| 36 | Rim Rv | CTCATCAGGCCATCGAGTTC |
| 37 | rut Fw | GAAACGTTCAGGTGTCGGAG |
| 38 | rut Rv | CGATTCGTTGGCTGATGGTG |
| 39 | dnc Fw | CAAACGCATGCTCAACAAGG |
| 40 | dnc Rv | GATGGCAAGTCGAACTCCTG |
| 41 | amn Fw | CAAATGTTCCGAGCGTCCAC |
| 42 | amn Rv | GCAGCGGTTCCGTTTGATAC |
| 43 | CaMKII Fw | GGGAGCCATACTTACGACAATG |
| 44 | CaMKII Rv | TCGGTTGATTCTTTGACCTGTG |
| 45 | Orb2 Fw | AACTGTGACTCCCTCTCCAG |
| 46 | Orb2 Rv | TTGCGCAGACTAACTTCGTC |
| 47 | 1tub23cf q-PCR Fw | TAATGGGCTCGGTCTACTCC |
| 48 | 1tub23cf q-PCR Rv | CCGCGATACTTCTCTCCAC |
| | RNApol2_Q q-PCR Fw | CCGCGATACTTCTCTCCAC |
| 49 | RNApol2_Q q-PCR Rv | GACCAGCTAGGCGACATTC |

For each condition, a minimum of 5 biological replicates and two technical replicates were analysed. Differential gene expression was calculated using the 2ΔΔCt method. The average Ct value for each sample was calculated and subtracted from the geometric mean Ct value of the reference genes RNApol2 and 1tub23cf in order to calculate the ΔCt value. *t* test (GraphPad Prism version 5.00 for Windows) was employed for calculations of *P*-values.

### mRNAsequencing of fat body and fly heads.

The starting material for sequencing library preparation was total RNA. The samples were quantified using the Qubit^{®} RNA BR Assay kit (Thermo Fisher Scientific), and RNA integrity was estimated with the RNA 6000 Nano Bioanalyzer 2100 Assay (Agilent). The RNA-Seq libraries were constructed using the KAPA Stranded mRNA-Seq Illumina Platforms Kit (Roche), following the manufacturer's recommendations. The size and quality of the libraries were assessed using a High Sensitivity DNA Bioanalyzer assay (Agilent). The libraries were sequenced on a NovaSeq 6000 (Illumina) in paired-end mode, with a read length of 2x51bp, following the manufacturer's protocol for dual indexing. Image analysis, base calling, and quality scoring of the run were processed using the manufacturer's software, Real Time Analysis (RTA 3.4.4). RNA-seq reads were mapped Drosophila melanogaste reference genome (BDGP6.32) with STAR/2.7.8a (ref1) with ENCODE parameters (A. Dobin, et al.2013. Bioinformatics. 29, 15-21). Gene quantification was performed with RSEM/1.3.0 (B. Li, 2011. BMC Bioinformatics. 12 (2011), doi:10.1186/1471-2105-12-323) with default parameters and using ensembl109 annotation. Only protein-coding genes that were expressed >1 cpm in at least 10 samples were considered.

### Mice experiment

### Animals.

Male wild-type C57BL/6J (Charles River, France) 8-week-old mice were housed individually in controlled laboratory conditions (21 ± 1ºC, 55 ± 10%) with illumination at 12-h cycles (lights on at 7:30 am and off at 7:30 pm). Animals were fed *ad libitum* for 8 weeks either with a normal chow diet [ND (~ 3.514 kcal/kg: 10% fat, 66% carbohydrate and 24% protein); n=23] or a high-fat diet [HFD (-5.228 kcal/kg: 60% fat, 16% carbohydrate and 24% protein); n=25]. ND and HFD were obtained from Altromin Spezialfutter GmbH & Co. (KG, Lage, Germany); references C1090-10, and C1090-60, respectively. Three days after the start of the diet, weight-matched mice of each group (ND or HFD) were divided into two groups and injected intravenously via tail vein with 200 µL of either the AAV8-RSV-GFP-Adp-miRNASLC18A2 vector containing 1·10^12 GC (Figure 1c) in phosphate-buffered saline or with saline. AAV vector was produced by the Viral Production Unit, UAB (VPU) (https://www.viralvector.eu/). The AAV2/8-RSV-GFP-Adp-miRNASLC18A2 vector expresses the green fluorescent protein (GFP) as well as a miRNA designed to downregulate SLC18A2 gene expression.. Adipose selectivity was enhanced by incorporating a proximal promoter and distal enhancer of the adiponectin gene into the AAV vector. In order to decrease vector expression in the liver, this AAV incorporates 8 copies of the liver-specific miR-122T into the 3'UTR of the expression cassette (S. M. O'neill, et al. 2014. Gene Ther. 21, 653-661) (Figure 1c). Bodyweight and food intake were monitored weekly for the entire protocol. All experiments were performed during the light phase. Mice were sacrificed by decapitation, and mesenteric white adipose tissue (mWAT) was collected and frozen with dry ice. All procedures were conducted in strict accordance with the guidelines of the European Communities Directive 86/609/EEC regulating animal experimentation and were approved by the local ethical committee (Comitè Ètic d'Experimentació Animal-Parc de Recerca Biomèdica de Barcelona, CEEA-PRBB, agreement N°9687)

### Experimental procedure.

Behavioral tasks aimed to assess short- and long-term memory (novel object recognition in V-maze), and locomotor activity (horizontal and vertical activity assessment) were performed chronologically as indicated in (Figure 1d,) using standardized tests as follows: *Novel object recognition test (V-MAZE).* Object-recognition memory was assayed in a V-shaped maze (30 cm long x4.5 cm wide *×* 15 3 cm height each corridor) (74, 75). On day 1 of the test, mice were habituated for 9 min to the maze. Then, 24h later, mice were put back in the maze for 9 min, two identical objects were presented, and the time that the mice spent exploring each object was recorded. The mice were again placed in the maze 3 h (30 days after the AAV injection to assess short-term memory) or 24h (37 days after the AAV injection to assess long-term memory) later for 9 min, with one of the familiar objects replaced with a novel object. The total time spent exploring each of the two objects (novel and familiar) was computed. A discrimination index was calculated as the difference between the time spent exploring the novel object divided by the total time exploring both objects. A higher discrimination index is considered to reflect greater memory retention for the familiar object.

*Locomotion.* On day 46 after AAV injection, mice underwent a 1-h locomotion test in individual locomotor activity boxes (10.8×20.3×18.6 cm, Imetronic, Pessac, France) equipped with infrared sensors to detect locomotor activity and an infrared plane to detect rearing.

*Statistical analysis.* The number of animals (n) in each experimental condition is indicated in figure legends. All graphs and statistical comparisons were performed using GraphPad Prism software version 8.0 for Mac (GraphPad Software, San Diego, CA, USA). To test whether the data were normally distributed, the Kolmogorov-Smirnov test was applied. Comparison of means between different groups was performed using two-way ANOVA followed by Newman-Keuls multiple comparisons test as post-hoc test. Three-way ANOVA was performed when required to test the evolution over time. Results are expressed as individual values with the mean ± SEM or mean ± SEM, specified in the figure legend. A p-value <0.05 was used to determine statistical significance.

*RNA expression.* RNA purification was performed using RNeasy Lipid Tissue Mini Kit (Qiagen, Hilden, Germany) and concentrations were measured using a Nanodrop ND-1000 Spectrophotometer (Thermo Fisher Scientific, Waltham, MA). Integrity was checked by Agilent Bioanalyzer (Agilent Technologies, Palo Alto, CA). Total RNA was reversed transcribed to cDNA using High Capacity cDNA Archive Kit (Applied Biosystems, Darmstadt, Germany). Gene expression was assessed by real time PCR using a LightCycler^{®} 480 Real-Time PCR System (Roche Diagnostics SL, Barcelona, Spain), using SYBR Green^{®} technology suitable for relative genetic expression quantification. The RT-PCR reaction was performed in a final volume of 10 µl. The cycle program consisted of an initial denaturing of 10 min at 95 °C then 40 cycles of 15 s denaturizing phase at 95 °C and 1 min annealing and extension phase at 60 °C. A threshold cycle (Ct value) was obtained for each amplification curve and then a ΔCt was first calculated by subtracting the Ct value for endogenous control from the Ct value for each sample. Fold changes compared with the endogenous control were then determined by calculating 2-ΔCt. β-actin (Actb) was used as housekeeping. Actin (Actb) and green fluorescent protein (GFP) were analysed by SYBR green technology using the following primer sets:
Actb (F: 5'-GATGTATGAAGGCTTTGGTC-3' (SEQ ID NO: 31), R: 5'-TGTGCACTTTTATTGGTCTC-3' (SEQ ID NO: 32)); and
GFP (F: 5'-CAACAGCCACAACGTCTATATCATG-3' (SEQ ID NO: 33), R: 5'-ATGTTGTGGCGGATCTTGAAG-3' (SEQ ID NO: 34)).

### Protein Analysis.

Adipose tissue samples (100mg) were homogenized using Radioimmunoprecipitation (RIPA) lysis buffer system (sc-24948, Santa Cruz Biotechnology, CA) at 4ºC for 30 min. Cellular debris were eliminated by centrifugation at 13,000g for 15min (4ºC). Protein concentration was determined using the RC/DC Protein Assay (Bio-Rad Laboratories, Hercules, CA). For Western Blot analysis, samples were resolved by SDS-PAGE and transferred to a PVDF membrane (BIO-RAD Laboratories, California, USA). Membrane was exposed overnight at 4ºC to primary antibody VMAT2/SLC18A2 (EB06558) at 1/250 dilution (VWR International EuroLab, SL) diluted in 2% BSA - 1x TBS containing 0.1% Tween-20, following the recommendations of the manufacturer. After secondary antibody incubation (Anti-Goat HRP), signal was detected using enhanced chemiluminescence HRP substrate (Millipore) and analysed with a luminescent image analyser ChemiDoc MP Imaging System (BIO-RAD Laboratories, California, USA). TGX Stain-Free gels (#4568096, BIO-RAD Laboratories, California, USA) were used as protein loading control.

### Statistical analysis.

Differential expression gene analyses were performed on gene counts using the "limma" R package (M. E. Ritchie, et al. 2015. Nucleic Acids Res. 43, e47). First, low expressed genes were filtered, so that only genes with more than 10 reads in at least 2 samples were selected. RNA-seq data were then normalized for RNA composition using the trimmed mean of M-value (TMM) as implemented in edgeR package (M. D. Robinson, et al.2010. Bioinformatics. 26, 139-140) . Normalized counts were then converted to log2 count per million (logCPM) with associated precision weights to account for variations in precision between different observations using the "voom" function with donor's age, BMI, sex, and education years as covariates. A robust linear regression model adjusted for the previous covariates was then fitted to the data using the "ImFit" function with the option method = "robust", to limit the influence of outlying samples. Finally, an empirical Bayes method was applied to borrow information between genes with the "eBayes" function. *P*-values were adjusted for multiple comparisons using the Benjamini-Hochberg procedure for False Discovery Rate (pFDR). A cut-off for the pFDR<0.05 was used a threshold for statistical significance. The functional roles of differentially expressed genes were characterized using over-representation analyses based on the Reactome and Wikipathways databases using ConsensusPathDB (A. Kamburov, et al. 2013. Nucleic Acids Res. 41, D793-D800). Pathway significance was assessed using a hypergeometric test and a Storey procedure (qvalue) was applied for multiple testing correction. Statistical significance was set at qvalue<0.1. For differentially expressed genes simultaneously in the VAT and SAT of the discovery and validation cohorts, data were further analysed using functional gene-gene interaction networks mapping genes to the Search Tool for Retrieval of Interacting Proteins/Genes (STRING) database (which integrates known and predicted protein/gene interactions) (D. Szklarczyk, et al. 2019. Nucleic Acids Res. 47, D607-D613) . Then, functional local clusters in the interaction network were determined using a Markov Cluster algorithm (MCL) with an inflation parameter = 1.4. Active interacting sources including text mining, experiments, databases, co-expression, and co-occurrence and an interaction score > 0.4 were used to construct the interaction networks. Additionally, we integrated the information provided from differential expression analysis, gene-gene interaction networks, and pathway over-representation analysis using the R package "pathfinder" with default parameters (E. Ulgen, et al.2019. Front Genet. 10, 858) . First, significant genes were mapped onto a STRING gene-gene interaction network. Then, active subnetworks of interconnected genes (including genes that are not significant themselves but connect significant genes) in this gene-gene interaction network were identified. Finally, separate pathway over-representation analyses based on the Reactome databases were performed for each active subnetwork using the significant genes in each of the active subnetworks and genes in the PIN as the background genes. Pathway statistical significance was set at pFDR<0.05. Significantly enriched pathways were clustered via hierarchical clustering. Genes in each pathway were used to calculate the pairwise kappa statistics, a chance-corrected measure of co-occurrence between pathways. The distance matrix 1- kappa statistic was used for agglomerative hierarchical clustering and a threshold of 0.35 for the kappa statistics was used to define strong relation. Partial Spearman's correlation analysis controlling for age, BMI, sex, and education level was used to determine the correlation between circulating levels of genes and the neurocognitive tests scores. As it works by obtaining the residuals of the ranked variables after removing the effect of the ranked covariates, scatter and violin plots were generated with the ranked residuals of the model adjusting for selected covariates. Non-parametric monotonic trends according the genes tertiles or quintiles for the selected cognitive tests were assessed by the Mann-Kendall trend test.

### II. RESULTS

### Adipose tissue gene expression is linked to several cognitive domains

To gain novel insights into the fat-brain axis, we performed a transcriptomics analysis (RNA-seq) of visceral adipose tissue (VAT) from 17 subjects with severe obesity (BMI > 35 mg/kg²) aged 28-60 years. Ten different cognitive tests assessed the functional performance of three cognitive domains: memory, executive function and attention. The tests and their interpretation are shown in methods.

Following normalization by trimmed mean of M-value (TMM) and calculating weights to account for variations in precision between observations, we fitted robust linear regression models with empirical Bayes moderation of the standard errors to identify gene transcripts associated with each cognitive test score controlling for age, sex, BMI and years of education, all confounding factors know to affect cognitive function. There are other lifestyle-related factors known to cause or contribute to cognitive function such as physical activity, diet or alcohol use . However, we found no significant associations of any of the 10 cognitive tests with physical activity, macronutrients, alcohol intake, glycemia or HbA1c. It worth nothing that all subjects had morbid obesity.

We observed significant associations with gene transcripts in all cognitive tests at pFDR<0.05. Among these genes, *NUDT2, AMPH, UNC5B* and OAT were those with most significant associations across the different cognitive domains (significant in 7, 6, 6 and 8 cognitive tests at pFDR<0.05, respectively). The attributed action of the proteins encoded by these genes was concordant with their function in the central nervous system but their expression in adipose tissue in association with cognition is unprecedented. Variants in the *NUDT2* gene have been recently associated to intellectual disability. *AMPH* encodes for a protein highly concentrated in presynaptic terminals , and is one of the six proteins altered in the cerebrospinal fluid of patients with mild cognitive impairment and Alzheimer's disease . *Amph* in *Drosophila* is an important postsynaptic protein that regulates the location of key components of glutamatergic signalling and dendrite dynamics, maturation and synaptogenesis . *OAT* is the gene that encodes for the enzyme ornithine aminotransferase, which plays a key role in transforming ornithine and arginine into glutamate and GABA, major excitatory and inhibitory neurotransmitters, respectively . In addition, genes involved in tryptophan metabolism such as *KYNU, QPRT,* and *INMT* were amongst the most significant genes associated with inhibitory control, attention, and executive function. Consistently, subjects with obesity had impaired memory and inhibitory control through alterations in tryptophan metabolism .

To facilitate interpretation and identify relevant adipose tissue pathways associated with cognitive function, we next performed over-representation analyses mapping significant genes to the Reactome and Wikipathways databases included in the ConsensusPathDB. Remarkably, we found an over-representation of pathways with important role in the central nervous system (CNS) associated with measurements of inhibitory control, working memory, immediate memory, and attention. Executive function was associated with axon guidance, nervous system development, synaptic vesicle pathway, and signalling by receptor tyrosine kinases. The neuronal system and the serotonin and dopamine neurotransmitter release cycles were the most significant pathways associated with immediate memory and attention. The axon guidance and the nervous system development were also over-represented in the inhibitory control and attention domains. Semaphorin signalling and interactions, which play an important role in adult neuronal plasticity , were associated with inhibitory control while alterations in complement activation were linked to executive function. We also found an over-representation of pathway involved in inflammation associated with the different cognitive domains. Hence, alterations in both notch signalling and complement activation were significantly associated with working memory.

### Adipose tissue gene expression was associated with several cognitive functions later in life

We next sought to validate these results performing a RNA-seq of both VAT and subcutaneous adipose tissue (SAT) in an independent longitudinal cohort of 22 subjects with severe obesity aged 23-57 years that underwent the same battery of cognitive tests. Patients from this cohort, underwent the same battery of cognitive tests that patients in the discovery cohort. However, in this case the cognitive testing was performed 2 to 3 years after the collection of adipose tissue samples. Once more, we did not find any significant association of physical activity, diet, alcohol intake, glucose, or Hba1c levels. Thus, models were adjusted for age, sex, BMI, and education years. We again found significant genes associated with all cognitive test at pFDR<0.05 both in VAT and SAT. Consistent with the previous results, enrichment analyses highlighted an over-representation of pathways with important roles in the central nervous system. Axon guidance, nervous system development, neuronal system and tryptophan were replicated as the pathways most significantly associated with attention, while semaphorin signalling was associated with inhibitory control. The activation of the complement system in VAT was again strongly associated with the performance in several cognitive domains. In SAT, pathways related to synaptic transmission, neuronal system, and one-carbon metabolism were associated with both long- and short-term memory. Complement activation in SAT was again amongst the pathways most significantly linked to attention.

The gene encoding for Ezrin (*EZR*) was one with most associations across the different cognitive domains in VAT (significant in 4 tests at pFDR<0.05), being the most significantly associated gene with the digit span forward score. In line with previous findings, complement-related genes were particularly and strongly associated with inhibitory control. This cognitive function was also associated with early immediate genes from the orphan nuclear receptor 4A family (*NR4A1, NR4A2*)*.* In particular, NR4A2was the most significant gene associated with the STROOPI test (which evaluates inhibitory control), while *NR4A1* was amongst the top genes associated with trail making test-B (executive function). In SAT, *NR4A1* and *NR4A2* were negatively associated with long-term memory, while the complement genes were particularly associated with attention. A gene from the nuclear receptor 4A family (*NR4A3*) had again the strongest negative association with inhibitory control. Finally, AMPH was the gene most significantly associated with digit span forward test and the total digit span test.

### Adipose tissue genes linked to cognitive function are involved in neuronal system and synaptic formation.

To gain further insights into the coincident associations between both cohorts, we performed an enrichment analysis considering only those genes significantly associated with at least one cognitive test in all three tissues (VAT from discovery, VAT and SAT from validation cohorts), a total of 188 coincident genes were found. Remarkably, the most over-represented pathways from the subset of genes included mostly pathways with key roles in synaptic function and inflammation (tryptophan metabolism and complement activation). Mapping these genes to the STRING database to construct a gene-gene interaction network, a cluster of highly interconnected genes mainly involved in neuronal system and synapse formation and maintenance (*DLG4, DLGAP1, DLG2, ATP1B1, HOMER1, NLGN3, SNCA, BCHE, RIMS1, SLC18A2, ADRB2, EZR, PODXL, NCAM2)* was disclosed.

To obtain a better picture of the associated pathways, we leveraged the interaction information from the gene-gene interaction network to identify distinct active subnetwork and then performed an active-subnetwork-oriented enrichment analysis on these subnetworks. Using this approach, we identified a total of 120 over-represented Reactome pathways (pFDR<0.05). To reduce complexity derived from this large number of pathways, we performed a hierarchical clustering to collapse redundant pathways and ease the identification of mechanisms relevant to cognition. Out of 53 clusters, the most significant one included pathways related to the neuronal system and synaptic function (*RIMS1, SLC18A2, DLG2, DLG4, DLGAP1, HOMER1, NLGN3, PTPRD, BCHE, KCNN3).* This cluster was strongly connected with three other cluster involved in the synthesis and metabolism of phospholipids, particularly phosphatidylinositols (PIPs) (*PIK3C2B, PIK3C2G, SYNJ1, PLEKHA6*)*,* the signalling by Rho GTPases (*ARHGAP23, DIAPH3, DLG4, RHOBTB1, SGO2, SRF*)*,* and the release of neurotransmitters (*RIMS1, SLC18A2*)*.*

To gain better insights into potential mechanisms linking adipose tissue to cognition, we next performed a multi-block PLS analysis to integrate these 188 adipose tissue genes with the plasma levels of metabolites measured by NMR, HPLC-ESI-MS/MS in positive and negative modes, and the circulating levels of cytokines and neurotoxic proteins (IL-6, IL-4, Tau, α-synuclein, Aβ-42). Remarkably, the expression levels of adipose tissue genes involved in phospholipid metabolism, such as *PIK3C2B, PLA2G6,* or *SYNJ1* had a strong association with several circulating phospholipids and lysophospholipids. Although lipids are essential to brain function and in particular phospholipids are crucial for synaptic plasticity, neurotransmission and memory , the brain can synthesize a limited number of lipids. However, plasma fatty acids can cross the BBB as non-esterified free fatty acids or esterified in lysophospholipids such as lysophosphatidylcholine (LPC) . Notably, some of the genes from the phospholipid metabolism cluster codified for phospholipase A2 (*PLA2G6*) that catalyzes the hydrolysis of the sn-2 position of glycerophospholipids to yield unesterified fatty acids and lysophospholipids. Hence, these phospholipids could be mediating the effects of adipose tissue on cognition.

We also identified three highly interconnected clusters of genes related to inflammatory response that included the complement cascade (*C4A, C4B*) and the IL-4, IL-10, IL-13 interleukin signaling (*IL18, TIMP1*)*.* We also identified a cluster containing the ATPase Na⁺/K⁺ transporting Subunit Beta 1 gene (*ATP1B1*)*.* Notably, the Na,K-ATPase signalling pathway requires the activation of phosphatidylinositol 3-kinase (PI3K) and we identified a cluster of pathways involved in PIPs metabolism strongly associated with cognition. Finally, the genes involved in the inflammatory cluster (*C4A, C4B, IL18, TIMP1*) had a strong positive association with the circulating levels of IL-6 and a strong negative association with the IL-4 levels in plasma.

Therefore, these adipokines could be released by the adipose tissue and mediate its effects on cognition.

Other significant clusters included the nuclear receptor transcription (*NR4A1, NR4A2, NR2F1)* and the metabolism of vitamins (*ABCC1, TCN2*)*,* particularly vitamin A *(RBP1, GPC4).* Recent studies have shown that *NR4A2* activation enhances long-term memory in young mice and ameliorates age-related memory impairments in old mice. Several studies have also suggested that water-soluble vitamins (C, E, Bs) may affect cognitive performance by reducing ROS generation and pro-inflammatory mediators such as NLRP3-inflammasome. Retinoic acid, the active form of vitamin A, is essential for regulating synaptic plasticity in regions of the brain involved in memory and learning. Notably, we found a strong positive association between the expression levels of genes involved in the metabolism of vitamins in the adipose tissue, in particular *TCN2* and *ABCC1,* and the circulating levels of retinoic acid. Notably, *RBP1,* which encodes for cellular retinol binding protein 1 (CRBP1) involved in the transport of retinol, had a strong negative association with the plasma levels of retinoic acid. Altogether, these results suggest the adipose tissue could modulate cognitive performance through the metabolism of vitamin A.

From the identified cluster of genes, *SLC18A2* and *RIMS1* (Regulating synaptic exocytosis 1) were those with the largest number of significant associations across the different cognitive domains in the three tissues analysed. *SLC18A2* encodes for VMAT2 (vesicle monoamine transporter member 2), a protein that transports cytoplasmic monoamine neurotransmitters (dopamine, norepinephrine, histamine, serotonin) into presynaptic vesicles and has been linked to the pathophysiology of different neuropsychiatric and neurological disorders. The *Drosophila* and mice *RIMS1* orthologues have shown to be a central component in the regulation of synaptic vesicle exocytosis and the neurotransmitter release. Mice lacking *Rim1α* show severely impaired learning and memory and *RIMS1* has recently been identified as the highest-ranked candidate gene in autism spectrum disorder.

### Morbid obesity modulates the direction of the associations between adipose tissue genes and cognition.

We sought to further validate these results using an additional cohort. Hence, we measured the expression levels of *EZR, UNC5B, NUDT2* and *NR4A2* in the VAT from a third independent validation cohort (ADIPOBRAIN cohort) consisting of n=40 patients with and without obesity aged 40-64 years old (Figure 2a) that underwent the Rey-Osterrieth Complex Figure (ROCF) test 4 to 10 years later. The ROCF is one of the top 10 tests used by neuropsychologists for the evaluation of visuospatial constructional ability, visual memory, and executive function. Consistent with our findings in the discovery cohort (including only patients with morbid obesity), the expression of *EZR* (Figure 2b) and *UNC5B* (Figure 2c) at baseline was strongly negatively associated with the ROCF copy scores in subjects with morbid obesity (BMI>35 kg/m²). Conversely, the expression of these genes was positively associated with executive function in subjects with a BMI<35 kg/m² (Figures 2g, 2g). The expression of *NUDT2*was positively associated with the ROCF copy performance in subjects with morbid obesity (Figure 2d) but not in subjects with BMI<35 kg/m² (Figure 2h), whereas we did not find any significant association with the expression of *NR4A2* (Figures 2e, 2i).

### Blood expression levels of key genes linked to cognitive performance

To further validate these findings and provide non-invasive biomarkers of cognitive function as an alternative to adipose tissue biopsies, we measured the expression of some of the most promising genes in the circulation of n=816 individuals from the general population aged ≥ 50 years taking part in the Aging Imageomics study (Figure 2j). Initially, we validated those genes with the largest number of associations across the different neuropsychological tests in the discovery cohort (*NUDT2, AMPH, UNCSB, OAT*) and validation cohort 1 (*EZR, NR4A2*)*.* In agreement with our findings, the circulating levels of *NUDT2* were negatively associated with executive function (Figure 2k), while the circulating expression of AMPHwas negatively associated with processing speed (Figure 2I) and memory (assessed by the total free recall; P*_{trend}*=0.02). Increased levels of *OAT* and *UNC5B* tended to be associated with delayed memory (Figure 2m) and executive function (Figure 2n). Finally, we also found consistent negative associations between the circulating expression of *EZR* and executive functioning (Figure 5o) and the quintiles of *NR4A2* with memory (*P*_{trend}=0.027).

### Downregulation of candidate genes in the fat body of Drosophila melanogaster improves cognition.

We next assessed the causal effect of the adipose tissue expression of these candidate genes (*AMPH, UNC5B, NUDT2, OAT, NR4A2, NR4A3 and EZR*) on cognitive function using the model organism *Drosophila melanogaster* (fruit flies). A main advantage of *Drosophila* is the possibility of manipulating gene expression in a tissue specific manner by means of the *UAS-GAL4* system (Figure 2p) (44). To evaluate *Drosophila* cognition, we used the courtship conditioning paradigm to measure associative learning and memory capabilities (T. S. Koemans, et al. 2017. Journal of Visualized Experiments. 2017, 55808). Male courtship behaviour is conditioned by the exposure to non-receptive pre-mated females resulting in a suppression of courtship after a learning process. Male courtship rates can be assessed at different times after training periods with pre-mated females. Learning (L) is assessed immediately after the training period and short-term memory is typically assessed one hour after training. The courtship index (Cl, the percentage of time spent on courtship during an 8-min interval) of trained (T) and of socially naive (N) male is used to calculate the Learning Index (LI). A higher LI is indicative of a better learning or short-term memory (Figure 2q).

We downregulated *Amph, unc-5, Datp, Oat, Hr38* and *Moe* (the well conserved orthologues of *AMPH, UNC5B, NUDT2, OAT, NR4A2*/*NR4A3,* and *EZR,* respectively) specifically in the fat body of *Drosophila,* which is the equivalent of the adipose tissue in humans. The fat body promoter line *w; C7-GAL4; UAS-Dcr-2* was crossed with at least one RNAi line targeting each gene of interest (Table S47). Learning capabilities were assessed immediately after training using the courtship conditioning assay. Downregulation of *unc-5, Datp, Hr38* and *Moe* lead to a significant decrease of learning capabilities compared to their corresponding genetic background controls in at least one of the RNAi lines tested (Figure 2r - 5u). Conversely, downregulation of *Amph* lead to an improvement of learning capabilities for one RNAi line (Figure 2v). Downregulation of *Oat* in the fat body was lethal for one RNAi line or did not affect learning captivities when using a second RNAi line (Figure 2w). Our findings demonstrate that specific downregulation of some of these genes in the fat body of *Drosophila* modulates learning, thereby suggesting a direct effect on cognition uniquely depending on its expression and/or function in fat body.

### Neurotransmitter release cycle-associated genes SLC18A2 and RIMS1 modulate cognition in mice adipose tissue or Drosophila fat body.

Among the genes associated with cognition in both VAT and SAT from the discovery and validation cohort1, the cluster of genes involved in the release of neurotransmitters (*RIMS1* and *SLC18A2*), had the largest number of significant associations across different cognitive domains. Therefore, we also validated these associations in the third validation cohort (ADIPOBRAIN). Again, we found that in subjects with morbid obesity (Figure 1a), the expression of *SLC18A2* at baseline was strongly negatively associated with the ROCF copy performance 4-10 years later, whereas it was positively associated in subjects with a BMI<35 kg/m² (Figure 1b).

To demonstrate a possible causal effect of adipose tissue *SLC18A2* on cognition, we studied two-preclinical models. In a mice model, we used an AAV2/8-RSV-GFP-Adp-miRNASLC18A2 vector that expresses a miRNA to selectively downregulate *slc18a2* gene expression in adipose tissue (Figure 1c). Eight-week old mice were fed a normal chow diet (ND, n=23) or a high-fat diet (HFD, n=25) for 8 weeks (Figure 1d). Three days after the start of the diet, each dietary group was divided into two groups and injected intravenously via tail vein either with the AAV2/8-RSV-GFP-Adp-miRNASLC18A2 vector (V) or with saline (S). The expression levels of green fluorescent protein (GFP) contained in the vector as a reporter gene was significantly increased in both inguinal and mesenteric white adipose tissue (iWAT and mWAT) of mice from the vectors-injected groups compared to the saline groups (Figures 3a, 3b), thereby indicating that the vector was able to reach the adipose tissue. We also found a significant decrease in the slc18a2 protein levels in the mWAT of virus-injected mice fed a HFD compared to their respective controls (Figure 3c, 3d). Four weeks post-injection, we assessed short- and long-term memory and locomotor activity. As expected, mice fed a HFD gained more weight in parallel to food intake compared with those fed a ND (Figure 1e, Figure 4a, 4b). Mice fed a HFD had lower short- and long-term memory than mice fed a ND (Figure 1f, 1g). Downregulation of *slc18a2* in the mice adipose tissue reversed the memory impairment induced by a HFD (Figure 1f, 1g). We observed no differences in the exploration time between groups (Figure 4c, 4d). Although we found a diet effect on locomotion, downregulation of *slc18a2* had no significant effect (Figures 4e-4h).

To further assess the role of adipose tissue *SLC18A* mRNA on cognition, we performed RNAi mediated knockdown of *Vmat* (the orthologue in *Drosophila*) using the *w; C7-GAL41 UAS-Dcr-2* promoter line for specific knockdown in the fat body (the equivalent of adipose tissue in humans). The courtship-conditioning paradigm was performed and short-term memory was assessed one hour after training. Males of *Vmat*-RNAi1were able to supress courtship significantly better than the corresponding genetic background control flies (Figure 1h). Consequently, the LI was significantly increased (*Vmat-RNAi1*: LI = 0.577, Control 2: LI = 0.366, *P*=0.0064), indicating that *Vmat-RNAi1* presented more short-term memory Figure 1h). Next, we performed RT-PCR to prove that *Vmatwas* specifically knocked down in the fat body but not in the fly head (Figure 1i), indicating that when decreasing *Vmat* expression exclusively in the fat body flies are able to learn better. To unravel a potential mechanism through which *Vmat* expression in the fat body could influence cognition, we performed RT-PCR to evaluated the expression of *rutabaga(rut), dunce (dnc), amnesiac (amn), homer, CAMKII* and *orb2* in *Drosophila* heads. These genes are part of the cyclic AMP (cAMP) pathway, which have been demonstrated to influence learning and short-term memory in the courtship conditioning assay. RNAi mediated knockdown of *Vmat* in the fat body lead to a significant increase in memory related genes (*rut*, *dnc, caMKII, and orb)* in the fly head (Figure 1j). To further validate our results, we used an independent RNAi line against *Vmat* (Vmat-RNAi2). When downregulating Vmat in the fat body with RNAi2 we observed a significant increase in short-term memory (*Vmat-RNAi2*: LI = 0.650, Control 1: LI = 0.437, *P*=0.002;) (Figure 5a) and a significant overexpression of *dnc, CAMKII and orb2 in the fly head* (Figure 5b), confirming our previous data.

In the third validation cohort, VAT *RIMS1* mRNA was also evaluated. In line with the results obtained for *SLC18A2,* the expression of *RIMS1* was negatively associated with ROCF in subjects with severe obesity (Figure 6a), but positively in subjects with a BMI<35 kg/m² (Figure 6b). To prove a possible causal effect, we assessed learning in *Drosophila* after downregulation or overexpression of *Rim* (the well-conserved orthologue of *RIMS1*) in the fat body. A significant decrease in CI after learning was observed in two independent RNAi lines and corresponding genetic background controls, but we did not observe significant differences in the LI (Figures 5c, 5d).

However, when *Rim* was overexpressed (*UAS-Rim*) in the fat body, males were able to suppress courtship significantly better than the corresponding genetic background control flies (Figure 7co), resulting in a significant increase in the LI (*UAS-Rim:* LI = 0.539, Control 3: LI = 0.237, *P*=0.0001) (Figure 6d). Our results indicate that flies that overexpress *Rim* exclusively in the fat body are able to learn better. Next, we used RT-PCR to prove that *Rim* was uniquely targeted in the fat body but not in the brain (Figure 6d). Again, learning improvement was exclusively promoted by *Rim* altered expression in the fat body and not due to a "leakage" of the RNAi in the brain.

To elucidate the potential mechanisms underlying the overexpression of *Rim* in learning, we performed an mRNA-seq in the fat body and fly heads of *UAS-Rim* flies that overexpressed *Rim* specifically in the adipocytes. We identified the differentially expressed genes between the flies overexpressing *Rim* and the corresponding genetic backgrounds in the fat body and fly heads. In the fat body, consistent with our results in humans, an enrichment analysis based on Reactome identified an over-representation of pathways involved in vitamin metabolism and lipid metabolism, including the hydrolysis of LPC, and the glutamate neurotransmitter release cycle. It also identified genes involved in other pathways involved in biological oxidations and cytochrome P450 (*Cyp4e1*, *Cyp4ac2, Cyp4ac3, Cyp4p1, Cyp4p2*)*.* Notably, a gene ontology (GO) enrichment analysis identified mainly biological processes participating in neurotransmitter regulation, in particular the metabolism of biogenic amines (dopamine, serotonin, norepinephrine), which is strongly consistent with our findings in humans. At the molecular function level, we identified several functions involved in monooxygenase and oxidoreductase activity (). Notably, we also identified several functions involved in the phosphatidylinositol transfer, and phospholipid transfer and transport. These results were replicated in the fly heads. We found again an over-representation of pathways involved in the metabolism of lipids (particularly phospholipids and the hydrolysis of LPC), vitamins and biological oxidations. An analysis based only on up-regulated genes also identified alterations in DNA metabolism, while a gene ontology analysis identified once more molecular functions related to monooxygenase and oxidoreductase activity.

Overall, we have demonstrated that overexpression of *Rim* uniquely in *Drosophila* fat body is sufficient to promote an increment in learning, and that the effect of *Rim* overexpression in the fat body can mediate changes at the transcriptional level in the adipose tissue itself and in peripheral tissues as the brain modulating pathways associated with neuronal functioning, cellular oxidative balance and detoxification and genes from the phospholipid metabolism.

Altogether, these results suggest that altering *Vmat* or *Rim* expression in the fat body mediates changes in gene expression in the head (brain) throughout a putative fat body-to-brain axis.

### III. DISCUSSION

While the brain regulates adipose tissue performance, we here show that the reverse is also plausible. We found that the expression of genes linked to axon guidance, nervous system development, neuronal system, tryptophan and inflammation activity in human adipose tissue were all linked to cognition in three different cohorts of subjects.

Downregulation of *Vmat* in the fat body of two different lines of *Drosophila* also led to improved short-term memory. Overexpressing *Rim* (the orthologue of Rims1) exclusively in the fat body also led to an increment in learning abilities in *Drosophila.* The altered expression of other genes linked to cognition in humans (*AMPH, UNC5B, NUDT2, NR4A2*/*NR4A3,* and *EZR)* in the fat body of *Drosophila* again modified cognition of the latter.

In the search of more accessible biomarkers, we evaluated the expression of most of these genes in PBMC. We here show that the expression of genes in PBMC that were most associated with cognition in adipose tissue (*NUDT2, AMPH, UNC5B, OAT, EZR, NR4A2)* were also linked to cognitive traits in a cohort of 816 subjects.

## Claims

1. An *in vitro* method for diagnosing a cognitive impairment in a subject, comprising
(i) determining the expression levels of a gene specifically expressed in adipose tissue, or of a fragment thereof, in a biological sample isolated from the subject, wherein
▪ said gene is:
- SLC18A2 (Solute Carrier Family 18 Member A2) gene, or
- RIMS1 (Regulating Synaptic Exocytosis 1) gene, or
- NUDT2 (Nudix hydrolase 2) gene, or
- AMPH (Amphiphysin) gene, or
- UNC5B (Unc-5 netrin receptor B) gene, or
- OAT (Ornithine Aminotransferase) gene, or
- EZR (Ezrin) gene, or
- NR4A2 (Nuclear Receptor subfamily 4, group A, member 2) gene, and
▪ the biological sample isolated from the subject is blood or adipose tissue biopsy; and
(ii) comparing the expression levels obtained in (i) with the expression levels of said gene in a control sample, wherein
- If the expression levels of SLC18A2 gene are increase with respect to the ones in the control sample, then the subject suffers from a cognitive impairment; or
- If the expression levels of RIMS1 gene are decrease with respect to the ones in the control sample, then the subject suffers from a cognitive impairment; or
- If the expression levels of NUDT2 gene are decrease with respect to the ones in the control sample, then the subject suffers from a cognitive impairment with respect to the ones in the control sample, then the subject suffers from a cognitive impairment; or
- If the expression levels of AMPH gene are decrease with respect to the ones in the control sample, then the subject suffers from a cognitive impairment; or
- If the expression levels of UNC5B gene are decrease with respect to the ones in the control sample, then the subject suffers from a cognitive impairment; or
- If the expression levels of OAT gene are decrease with respect to the ones in the control sample, then the subject suffers from a cognitive impairment; or
- If the expression levels of EZR gene are decrease with respect to the ones in the control sample, then the subject suffers from a cognitive impairment; or
- If the expression levels of NR4A2 gene are decrease with respect to the ones in the control sample, then the subject suffers from a cognitive impairment.

2. An *in vitro* method for monitoring the response to a therapy directed to improve the cognitive function in a subject suffering from cognitive impairment, comprising
(i) determining the expression levels of a gene specifically expressed in adipose tissue, or of a fragment thereof, in a biological sample isolated from the subject after the administration of the therapy to the subject, wherein
▪ said gene is SLC18A2 gene, RIMS1 gene, NUDT2 gene, AMPH gene, UNC5B gene, OAT gen, EZR gene or NR4A2 gene, and
▪ the biological sample isolated from the subject is blood or adipose tissue; and
(ii) comparing the expression levels obtained after the therapy with the expression levels of said gene in a control sample, wherein
- If the expression levels of SLC18A2 gene after the therapy are lower than the control sample, then response of the subject to the therapy is positive; or
- If the expression levels of RIMS1 gene after the therapy are higher than the control sample, then response of the subject to the therapy is negative; or
- If the expression levels of NUDT2 gene after the therapy are similar to the ones in the control sample, then response of the subject to the therapy is positive; or
- If the expression levels of AMPH gene are similar to the ones in the control sample, then response of the subject to the therapy is positive; or
- If the expression levels of UNC5B gene are similar to the ones in the control sample, then response of the subject to the therapy is positive; or
- If the expression levels of OAT gene are similar to the ones in the control sample, then response of the subject to the therapy is positive; or
- If the expression levels of EZR gene are similar to the ones in the control sample, then response of the subject to the therapy is positive; or
- If the expression levels of NR4A2 gene are similar to the ones in the control sample, then response of the subject to the therapy is positive.

3. The *in vitro* method according to claim 1 or 2, wherein the cognitive impairment is Mild Cognitive Impairment (MCI), Age-related Cognitive Decline (ARCD) or Age-Associated Memory Impairment (AAMI).

4. The *in vitro* method according to any one of claims 1 to 3, wherein the subject is a subject having obesity or overweight, preferably, the subject is a human subject having a body Mass Index (BMI) equal or higher than 25 kg/m², preferably, equal or higher than 30 Kg/m².

5. The *in vitro* method according to any one of claims 1 to 4, wherein determining the expression levels of the gene comprises measuring the level of cDNA, mRNA and/or the protein encoded by said gene.

6. Use of a kit (i) for the *in vitro* diagnosis of a cognitive impairment in a subject, or (ii) for monitoring the response of a subject to a therapy directed to improve the cognitive function in a subject suffering from cognitive impairment, wherein the kit comprises the reagents needed to determine the expression levels of one or more genes specifically expressed in adipose tissue, or of a fragment of said genes, wherein said genes are selected from the list consisting of the SLC18A2 gene, RIMS1 gene, NUDT2 gene, AMPH gene, UNC5B gene, OAT gen, EZR gene, NR4A2 gene, and combinations thereof.

7. Use according to claim 6, wherein the subject is a subject having obesity or overweight, preferably, the subject is a human subject having a body Mass Index (BMI) equal or higher than 25 kg/m², preferably, equal or higher than 30 Kg/m².

8. Use according to claim 6 or 7, wherein the reagents needed to determine the expression levels of the genes are
- a nucleic acid that specifically hybridises the gene, or
- antibodies that specifically recognise the protein encoded by the gene.

9. Use according to claim 8, wherein the nucleic acid that specifically hybridises the gene are primers or probes.

10. Use according to claim 9, wherein the primers are SEQ ID NO: 17, SEQ ID NO: 18, SEQ ID NO: 19, SEQ ID NO: 20, SEQ ID NO: 21, SEQ ID NO:22, SEQ ID NO: 23, SEQ ID NO: 24, SEQ ID NO: 25, SEQ ID NO: 26, SEQ ID NO: 27, SEQ ID NO: 28, SEQ ID NO: 29 and/or SEQ ID NO: 30.

11. A composition comprising a modulating agent of the adipose tissue gene expression levels for use in the treatment of a cognitive impairment in a subject, wherein the adipose tissue gene is SLC18A2 gene, RIMS1 gene, NUDT2 gene, AMPH gene, UNC5B gene, OAT gen, EZR gene or NR4A2 gene.

12. The composition for use in the treatment of a cognitive impairment according to claim 11, wherein the subject is a subject having obesity or overweight, preferably, the subject is a human subject having a body Mass Index (BMI) equal or higher than 25 kg/m², preferably, equal or higher than 30 Kg/m².

13. The composition for use in the treatment of a cognitive impairment according to claim 11 or 12, wherein the modulating agent of the gene expression levels is a miRNA or an iRNA that specifically hybridises the gene, or an antibody that specifically recognises the protein encoded by the gene.

14. The composition for use in the treatment of a cognitive impairment according to any one of claims 11 to 13, wherein the cognitive impairment is Mild Cognitive Impairment (MCI), Age-related Cognitive Decline (ARCD) or Age-Associated Memory Impairment (AAMI).
